# EUROPÄISCHE PATENTANMELDUNG

(11) **EP 3 424 968 A1**
(43) Veröffentlichungstag der Anmeldung: **09.01.2019**
(21) Anmeldenummer: 17179287.2
(22) Anmeldetag: 03.07.2017
(51) Int. Cl.: C08G 18/67, C08G 18/76, C08G 18/24, C08L 75/16

(54) **URETHANMETHACRYLAT-VERBINDUNGEN UND DEREN VERWENDUNG**

(71) Anmelder: HILTI Aktiengesellschaft, 9494 Schaan (LI)
(72) Erfinder: Gnaß, Beate, 86368 Gersthofen (DE); Bunzen, Jens, 86159 Augsburg (DE); Nickerl, Georg, 86911 Dießen am Ammersee (DE)
(74) Vertreter: Hilti Aktiengesellschaft Corporate Intellectual Property

(57) **Zusammenfassung**

Es werden niedrigviskose Urethanmethacrylat-Verbindungen als Backbone-Harz, deren Verwendung in Reaktivharzen, insbesondere zur Erniedrigung der Viskosität solcher Mischungen enthaltenden Reaktivharzen und damit der Auspresskräfte von daraus hergestellten Reaktivharzkomponenten sowie zur Erhöhung der Leistungsfähigkeit solcher Verbindungen enthaltenden Reaktivharze und daraus hergestellten Reaktivharzkomponenten beschrieben. Des Weiteren wird die Verwendung dieser Verbindungen und deren Reaktivharzkomponenten zu baulichen Zwecken, insbesondere zur chemischen Befestigung beschrieben.

## Beschreibung

Die Erfindung betrifft niedrigviskose Urethanmethacrylat-Verbindungen als Backbone-Harze, deren Verwendung in Reaktivharzen, insbesondere zur Erniedrigung der Viskosität solcher Verbindungen enthaltenden Reaktivharzen und damit der Auspresskräfte von daraus hergestellten Reaktivharzkomponenten. Des Weiteren betrifft die Erfindung die Verwendung dieser Reaktivharze und deren Reaktivharzkomponenten zu baulichen Zwecken, insbesondere zur chemischen Befestigung.

Die derzeit eingesetzten radikalisch härtbaren Befestigungsmassen basieren auf ungesättigten Polyestern, Vinylesterurethanharzen und Epoxyacrylaten. Es handelt sich dabei meist um Zweikomponenten-Reaktivharz-Systeme, wobei eine Komponente das Harz (sogenannte Komponente (A)) und die andere Komponente (Komponente (B)) das Härtungsmittel enthält. Weitere Bestandteile wie anorganische Füllmittel und Additive, Beschleuniger, Stabilisatoren und Reaktivverdünner können in der einen und/oder der anderen Komponente enthalten sein. Durch Vermischen der beiden Komponenten wird die Härtung der gemischten Komponenten in Gang setzt. Bei Verwendung der Befestigungsmassen zur Befestigung von Verankerungselementen in Bohrlöchern erfolgt die Härtung in den Bohrlöchern.

Eine solche Befestigungsmasse ist beispielsweise aus der DE 3940138 A1 bekannt. Diese beschreibt Befestigungsmassen auf Basis von cycloaliphatische Gruppen tragenden Monomeren, die zusätzlich ungesättigte Polyester- oder Vinylesterharze enthalten können. Derartige Befestigungsmassen weisen aber verhältnismäßig hohe Viskositäten auf, wodurch ihre Anwendung, vor allem für die chemische Befestigungstechnik beschränkt wird.

Auf Baustellen können relativ große Temperaturbereiche von beispielsweise -25°C bis +45°C auftreten, je nach Jahreszeit und/oder geographischer Lage. Daher kann die hohe Viskosität bei den eingangs beschriebenen härtbaren Befestigungsmassen und deren resultierendes thixotropes Verhalten bei der Anwendung zu Problemen führen. Daher werden große Anforderungen an den Einsatzbereich, insbesondere den Einsatz in verschiedenen Temperaturbereichen, derartiger Befestigungsmassen gestellt.

Zum einen sollte im niedrigen Temperaturbereich eine ausreichend niedrige Viskosität der Masse beim Auspressen gewährleistet werden, so dass die Masse einen nicht zu hohen Strömungswiderstand aufweist. Damit soll sichergestellt werden, dass die Massen beispielsweise mit einem Handdispenser verarbeitet, z.B. in das Bohrloch injiziert werden kann. Insbesondere bei der Verwendung von Statikmischern ist eine niedrige Viskosität für eine einwandfreie Vermischung der beiden Komponenten von Bedeutung.

Zum anderen sollte die Masse im höheren Temperaturbereich hinreichend standfest sein, so dass ein Nachlaufen der einzelnen Komponenten nach dem Entspannen des Dispensers verhindert wird sowie bei der Überkopf-Montage die Masse nicht aus dem Bohrloch herausläuft.

Ein weiteres durch Temperaturschwankungen bedingtes Problem ist, dass die radikalische Kettenpolymerisation nicht einheitlich abläuft. Die ausgehärtete Befestigungsmasse weist somit eine schwankende/unregelmäßige und häufig unzureichende Homogenität aus, was sich in Schwankungen der Lastwerte und häufig auch in generell niedrigen Lastwerten wiederspiegelt. Beispielsweise kann es bei Temperaturen unter 20°C durch eine Erhöhung der Viskosität zu einem frühzeitigen Erstarren der Befestigungsmasse kommen. Dadurch fällt der Umsatz bei der radikalischen Kettenpolymerisation wesentlich geringer aus, was zu einer Verringerung der Lastwerte beiträgt.

Da Temperaturschwankungen auf der Baustelle sich nicht vermeiden lassen, besteht nach wie vor ein Bedarf an Zweikomponenten-Reaktivharz-Systemen, welche sowohl bei hohen als auch bei niedrigen Temperaturen die Homogenität und die damit verbundene Reproduzierbarkeit der Lastwerte gewährleisten.

Um oben genannte Probleme zu adressieren, wird bei den auf dem Markt befindlichen Befestigungsmassen der Anteil an Reaktivverdünnern erhöht, was letztendlich zu einer Verringerung des Harzanteils in der Masse führt. Nicht selten beträgt der Anteil an Reaktivverdünnern mindestens 50%, bezogen auf das Reaktivharz.

Die Erhöhung des Anteils an Reaktivverdünnern führt allerdings auch zu einigen Nachteilen, die sich vor allem bei der Anwendung der Befestigungsmasse zur Befestigung von Verankerungsmittel in Bohrlöchern bemerkbar machen.

Ein erheblicher Nachteil ist, dass die Verringerung des Anteils an hochviskosem Harz, welches für die Leistungsfähigkeit der Masse wesentlich ist, die Leistungsfähigkeit der ausgehärteten Befestigungsmasse negativ beeinflusst.

Ein weiterer Nachteil ist ein größerer Schrumpf der Befestigungsmasse nach dem Aushärten, was zusätzlich die Leistungsfähigkeit der ausgehärteten Befestigungsmasse negativ beeinflussen kann. Dies wird darauf zurückgeführt, dass der Kontakt zwischen der ausgehärteten Befestigungsmasse und den beim Erstellen des Bohrlochs in der Bohrlochwandung gebildeten Hinterschnitten, die insbesondere bei der Verwendung von Schlagbohrmaschinen auftreten, deutlich verringert wird. Dies verhindert meist auch die Anwendung der Befestigungsmassen auf Basis radikalisch härtbarer Verbindungen in diamantgebohrten Löchern.

Ein weiterer Nachteil ist, dass sich je nach Art des Reaktivverdünners der Anteil an flüchtigen organischen Verbindungen (VOC; Volatile Organic Compounds) in den Massen erhöhen kann. Dies kann zu Ausdünstungen aus der Befestigungsmasse und/oder der Kartusche führen und gegebenenfalls zu einem daraus resultierenden Leistungsabfall der ausgehärteten Befestigungsmasse. Einige dieser Verbindungen können zudem auch gesundheitsgefährdend sein und/oder sind daher kennzeichnungspflichtig.

Die Anzahl an einsetzbaren Reaktivverdünner ist zudem gering, da es derzeit nur wenige verfügbare Reaktivverdünner auf dem Markt gibt. Die verfügbaren Reaktivverdünner tragen neben den radikalisch härtbaren funktionellen Gruppen keine oder nur eine sehr eingeschränkte Auswahl an anderen funktionellen Gruppen und haben daher oft nur wenig Einfluss auf die Eigenschaft der ausgehärteten Befestigungsmasse. Dies führt dazu, dass die Befestigungsmassen meist für bestimmte Anwendungen entwickelt werden, wie etwa bestimmte Temperaturbereiche, oder zur Anwendung in bestimmten Untergründen. Dies spricht für einen immensen Entwicklungsaufwand, um neue und breitere Anwendungen mit den Befestigungsmassen adressieren zu können.

Bisher werden Spezialprodukte, deren Formulierungen auf die speziellen Anwendungstemperaturen angepasst sind, hergestellt. Es gibt zwar Produkte, die für einen breiten Temperaturbereich gedacht sind, die jedoch über den gesamten Bereich gleiche Eigenschaften aufweisen. Gerade in den Randbereichen, d.h. bei niedrigen und bei hohen Temperaturen, muss entweder mit Beeinträchtigungen bei der Verarbeitbarkeit, bei der Aushärtung der Masse, oder bei den Eigenschaften der ausgehärteten Masse gerechnet werden. Es ist keine Befestigungsmasse bekannt, die einen sehr großen Temperaturbereich abdeckt, ohne Einbußen in den Randbereichen hinnehmen zu müssen.

Es besteht daher ein Bedarf an Befestigungsmassen deren Leistungsfähigkeit und Eigenschaften nicht durch den Einsatz von Reaktivverdünnern, sondern durch den Harzbestandteil beeinflusst werden kann.

Eine Aufgabe der vorliegenden Erfindung ist die Beeinflussung der Eigenschaften eines Reaktivharz-Masterbatches sowie eines daraus hergestellten Reaktivharzes, die allein auf die Struktur des Backbone-Harz zurückzuführen ist, nicht jedoch auf die Präsenz von zusätzlichen Verbindungen, wie z. B. Reaktivverdünner oder Additive. Hauptsächlich ist es Aufgabe der vorliegenden Erfindung die Eigenschaften eines Zwei- oder Mehrkomponenten-Reaktivharz-Systems mittels des enthaltenden Backbone-Harzes zu steuern. Insbesondere ist es Aufgabe der vorliegenden Erfindung Befestigungsmassen, wie beispielsweise Zwei- oder Mehrkomponenten-Reaktivharz-Systeme, bereitzustellen, deren Viskosität weniger von der Anwendungstemperatur der Befestigungsmasse abhängt, die insbesondere bei niedrigen Temperaturen, wie etwa unter 20°C, eine niedrige Viskosität aufweisen und damit die Bereitstellung von Reaktivharz-Systemen ermöglichen, die bei Anwendungstemperaturen unter 20°C, insbesondere bei Anwendungstemperaturen unter 10°C niedrigere Auspresskräfte aufweisen und damit anwenderfreundlicher sind als die herkömmlichen Befestigungssysteme.

Eine weitere Aufgabe der Erfindung ist die Bereitstellung einer Befestigungsmasse, welche niedrigere Auspresskräfte der Reaktivharz-Komponente und gleichzeitig höhere Lastwerte der ausgehärteten Befestigungsmasse aufweist als herkömmliche Massen.

Eine noch weitere Aufgabe der vorliegenden Erfindung ist die Bereitstellung einer Befestigungsmasse, welche hochgradig gesundheitsgefährdende Inhaltsstoffe in der Reaktivharz-Komponente vermeidet und gegebenenfalls auch kennzeichnungsfrei ist. Insbesondere ist es Aufgabe, den Anteil an Reaktivverdünnern in Reaktivharzen für die chemische Befestigung zu verringern, ohne auf deren Funktion bzw. Funktionen und positiven Auswirkungen auf die ausgehärtete Befestigungsmasse verzichten zu müssen.

Eine noch weitere Aufgabe der vorliegenden Erfindung ist die Bereitstellung einer Befestigungsmasse, welche sich durch gute Verarbeitbarkeit, Aushärteverhalten und durch geringen Schrumpf über eine große Temperaturspanne auszeichnet.

Diese Aufgaben werden durch die Verbindungen nach Anspruch 1 und deren Verwendung nach den Ansprüchen 5 bis 8, das Reaktivharz nach Anspruch 9 und die Reaktivharzkomponente nach Anspruch 10 gelöst.

Überraschender Weise wurde gefunden, dass durch die Verwendung bestimmter niedrigviskoser Urethanmethacrylat-Verbindungen als Backbone-Harz die Temperaturspanne, in der die Viskosität eines diese Verbindungen enthaltenden Reaktivharzes und einer daraus erhältlichen Reaktivharzkomponente von den Temperaturen weitestgehend wenig beeinflusst bleibt, groß ist.

Vorteilhafterweise ermöglicht die vorliegende Erfindung bei niedrigen Anwendungstemperaturen niedrige Auspresskräfte bei einem Reaktivharz-System im Vergleich zu den herkömmlichen Systemen. Durch den Einsatz von niedrigviskosen Urethanmethacrylat-Verbindungen als Backbone-Harz in Reaktivharzen ist es somit gelungen, die Auspresskräfte eines Reaktivharz-Systems sowohl bei 20°C als auch bei niedrigeren Temperaturen, beispielsweise bei Temperaturen unter 10°C, bevorzugt unter 5°C zu senken, ohne dass hierzu ein hoher Anteil an Reaktivverdünner erforderlich ist.

Ferner wurde gefunden, dass es möglich ist, durch den Einsatz bestimmter niedrigviskoser Urethanmethacrylat-Verbindungen den Anteil an Reaktivverdünnern in Reaktivharzen für die chemische Befestigung zu verringern, ohne auf deren Funktion bzw. Funktionen und positiven Auswirkungen auf die ausgehärtete Befestigungsmasse verzichten zu müssen, da der Anteil an Backbone-Harz erhöht werden kann. Hierdurch wird es zum einen möglich, die Lastwerte einer ausgehärteten Masse zu steigern, und zum anderen bei höheren Temperaturen, beispielsweise bei 80°C, bei gleichem Anteil an Backbone-Harz höhere Lastwerte zu erzielen.

Die Erfindung beruht auf der Erkenntnis, dass es möglich ist, die bisher in Befestigungsmassen eingesetzten höherviskosen Harze durch kleinere, niedrigviskose Backbone-Harze zu ersetzen, um den Anteil an Reaktivverdünner zu verringern, ohne auf deren Funktionalität verzichten zu müssen.

Zum besseren Verständnis der Erfindung werden die folgenden Erläuterungen zum Herstellungsverfahren für ein Reaktivharz und zur hierin verwendeten Terminologie als sinnvoll erachtet.

Das Herstellungsverfahren für ein Reaktivharz verläuft, hier erläutert am Beispiel eines MDI-basierten Urethanmethacrylates, typischerweise wie folgt:

### 1. Herstellung Backbone-Harz/Reaktivharz-Masterbatch

Methandiphenyldiisocyanat (MDI) und Hydroxypropylmethacrylat (HPMA) werden in Anwesenheit eines Katalysators und eines Inhibitors (dient dazu, das durch die Polymerisation gebildete Backbone-Harz zu stabilisieren, häufig auch Stabilisator oder Prozessstabilisator genannt) zur Reaktion gebracht. Hierbei entsteht das Backbone-Harz.

Das nach Ende der Reaktion erhaltene Reaktionsgemisch wird als Reaktivharz-Masterbatch bezeichnet. Dieses wird nicht weiter aufgearbeitet, d.h. das Backbone-Harz wird nicht isoliert.

### 2. Herstellung Reaktivharz

Zu dem Reaktivharz-Masterbatch werden nach Abschluss der Reaktion zum Backbone-Harz ein Beschleuniger-Inhibitor-System, d.h. eine Kombination aus einem oder mehreren zusätzlichen Inhibitoren und einem oder mehreren Beschleunigern sowie gegebenenfalls ein Reaktivverdünner gegeben.

Hierdurch erhält man das Reaktivharz.

Das Beschleuniger-Inhibitor-System dient dazu, die Reaktivität des Reaktiv-Harzes einzustellen, also den Zeitpunkt einzustellen, bis zu dem das Reaktiv-Harz nach Zugabe eines Initiators noch nicht vollständig ausgehärtet ist und bis zu welchem Zeitpunkt daher eine mit dem Reaktiv-Harz angemischte Dübelmasse nach dem Mischen mit dem Initiator verarbeitbar bleibt.

Der Inhibitor in dem Beschleuniger-Inhibitor-System kann gleich dem Inhibitor bei der Herstellung des Backbone-Harzes sein, wenn dieser auch dazu geeignet ist, die Reaktivität einzustellen, oder ein anderer Inhibitor sein, wenn er nicht beide Funktionen aufweist. 4-Hydroxy-2,2,6,6-tetramethyl-piperidinyl-1-oxyl (TEMPOL) etwa kann als Stabilisator und als Inhibitor zu Einstellung der Reaktivität eingesetzt werden.

### 3. Herstellung Reaktivharzkomponente

Für die Verwendung des Reaktivharzes für bauliche Zwecke, insbesondere für die chemische Befestigung, werden nach der Herstellung des Reaktivharzes ein oder mehrere anorganische Zuschlagstoffe, wie Additive und/oder Füllstoffe, gegeben.

Hierdurch wird die Reaktivharzkomponente erhalten.

Im Sinne der Erfindung bedeuten die verwendeten Begriffe:
- *"Backbone-Harz"* ein üblicherweise festes oder hochviskoses radikalisch polymerisierbares Harz, welches durch Polymerisation (z.B. nach Zugabe eines Initiators in Gegenwart eines Beschleunigers) härtet und in der Regel ohne Reaktivverdünner und ohne weiterer Reinigung vorliegt und damit Verunreinigung enthalten kann;
- *"Reaktivharz-Masterbatch"* das Reaktionsprodukt der Reaktion zur Herstellung des Backbone-Harzes, also eine Mischung aus Backbone-Harz, Reaktivverdünner und gegebenenfalls weiteren Bestandteilen der Reaktionsmischung;
- *"Reaktivharz"* eine Mischung aus Reaktivharz-Masterbatch, mindestens einem Beschleuniger und mindestens einem Inhibitor (auch als Beschleuniger-Inhibitor-System bezeichnet), mindestens einem Reaktivverdünner und gegebenenfalls weiteren Additiven; das Reaktivharz ist typischerweise flüssig oder viskos und kann zu einer Reaktivharzkomponente weiterverarbeitet werden; hierin wird das Reaktivharz auch als *"Harzmischung"* bezeichnet;
- *"Inhibitor"* einen Stoff, der eine unerwünschte radikalische Polymerisation während der Synthese oder der Lagerung eines Harzes oder einer Harz-haltigen Zusammensetzung unterdrückt (diese Stoffe werden in Fachkreisen auch als *"Stabilisator"* bezeichnet) bzw. der eine radikalische Polymerisation eines Harzes nach Zugabe eines Initiators (üblicherweise in Verbindung mit einem Beschleuniger) zeitlich verzögert (diese Stoffe werden in Fachkreisen auch als *"Inhibitor"* bezeichnet - die jeweilige Bedeutung des Begriffes erschließt sich aus dem Kontext);
- *"Beschleuniger"* ein Reagenz, welches mit dem Initiator eine Reaktion eingeht, so dass bereits bei niedrigen Temperaturen durch den Initiator größere Mengen an Radikalen erzeugt werden, oder welches die Zerfallsreaktion des Initiators katalysiert;
- *"Reaktivverdünner"* flüssige oder niedrigviskose Monomere und Backbone-Harze, welche andere Backbone-Harze oder den Reaktivharz-Masterbatch verdünnen und dadurch die zu deren Applikation notwendige Viskosität verleihen, zur Reaktion mit dem Backbone-Harz befähigte funktionelle Gruppen enthalten und bei der Polymerisation (Härtung) zum überwiegenden Teil Bestandteil der gehärteten Masse (z.B. des Mörtels) werden; Reaktivverdünner werden auch co-polymerisierbares Monomer genannt;
- *"Reaktivharzkomponente"* eine flüssige oder viskose Mischung aus Reaktivharz und Füllstoffen und optional weiteren Komponenten, z.B. Additiven; typischerweise ist die Reaktivharzkomponente eine der beiden Komponenten eines Zweikomponenten-Reaktivharz-Systems zur chemischen Befestigung;
- *"Initiator"* einen Stoff, der (üblicherweise in Kombination mit einem Beschleuniger) reaktionsinitierende Radikale bildet;
- *"Härterkomponente"* eine Zusammensetzung, die einen Initiator für die Polymerisation eines Backbone-Harzes enthält; die Härterkomponente kann fest oder flüssig sein und neben dem Initiator ein Lösungsmittel sowie Füllstoffe und/oder Additive enthalten; typischerweise ist die Härterkomponente neben der Reaktivharzkomponente die andere der beiden Komponenten eines Zweikomponenten-Reaktivharz-Systems zur chemischen Befestigung;
- "Mörtelmasse/*Befestigungsmasse"* die Zusammensetzung, die durch das Mischen der Reaktivharzkomponente mit der Härterkomponente erhalten wird und als solche direkt zur chemischen Befestigung verwendet werden kann;
- *"Reaktivharz-System"* allgemein ein System, das voneinander getrennt gelagerte Komponenten umfasst, so dass eine Härtung des in einer Komponente enthaltenen Backbone-Harzes erst nach dem Mischen der Komponenten erfolgt;
- *"Zweikomponenten-System"* bzw. *"Zweikomponenten-Reaktivharz-System"* ein Reaktivharz-System, das zwei voneinander getrennt gelagerte Komponenten, eine Reaktivharzkomponente (A) und eine Härterkomponente (B), umfasst, so dass eine Härtung des in der Reaktivharzkomponente enthaltenen Backbone-Harzes erst nach dem Mischen der beiden Komponenten erfolgt;
- *"Mehrkomponenten-System"* bzw. *"Mehrkomponenten-Reaktivharz-System"* ein Reaktivharz-System, das mehrere voneinander getrennt gelagerte Komponenten umfasst, unter anderem eine Reaktivharzkomponente (A) und eine Härterkomponente (B), so dass eine Härtung des in der Reaktivharzkomponente enthaltenen Backbone-Harzes erst nach dem Mischen aller Komponenten erfolgt;
- *"bauliche Zwecke"* jegliche Anwendung zur Erstellung und Instandhaltung bzw. - setzung von Bauteilen und Bauwerken, als Polymerbeton, als Beschichtungsmasse auf Kunstharzbasis oder als kalthärtende Straßenmarkierung; insbesondere die Verstärkung von Bauteilen und Bauwerken, beispielsweise Wände, Decken oder Böden, die Befestigung von Bauteilen, wie Platten oder Blöcken, z. B. aus Stein, Glas oder Kunststoff, an Bauteilen oder Bauwerken, etwa durch Kleben (bauliches Kleben) und ganz besonders die chemische Befestigung von Verankerungsmitteln, wie Ankerstangen, Bolzen oder dergleichen in Aussparungen, wie Bohrlöchern;
- *"chemische Befestigung"* eine (stoff- und/oder formschlüssige) Befestigung von Verankerungsmitteln, wie Ankerstangen, Bolzen, Bewehrungseisen, Schrauben oder dergleichen in Aussparungen, wie Bohrlöchern, insbesondere in Bohrlöchern in verschiedenen Untergründen, insbesondere mineralischen Untergründen, wie solche auf der Grundlage von Beton, Porenbeton, Ziegelwerk, Kalksandstein, Sandstein, Naturstein, Glas und dergleichen, und metallischen Untergründen, wie solche aus Stahl;
- *"aromatische Kohlenwasserstoffgruppe",* eine cyclische, planare Kohlenwasserstoffgruppe mit aromatischem System, die aufgrund ihres delokalisierten Elektronensystems energetisch günstiger als ihre nicht aromatischen Mesomere und deshalb chemisch stabiler sind (PAC, 1995, 67, 1307; Glossary of class names of organic compounds and reactivity intermediates based on structure (IUPAC Recommendations 1995) page 1319);
- *"aromatisches Diisocyanat,* dass die beiden Isocyanatgruppen direkt an ein aromatisches Kohlenwasserstoffgerüst gebunden sind;
- *"(Meth)acryl...*/*...(meth)acryl...",* dass sowohl die "Methacryl.../...methacryl... "- als auch die "Acryl.../...acryl..."-Verbindungen gemeint sein sollen; bevorzugt sind in der vorliegenden Erfindung "Methacryl.../...methacryl..."-Verbindungen gemeint;
- *"ein", "eine", "einer"* als Artikel vor einer chemischen Verbindungsklasse, z.B. vor dem Wort "Urethanmethacrylat", dass mindestens eine, d.h., eine oder mehrere unter diese chemische Verbindungsklasse fallende Verbindungen, z.B. verschiedene Urethanmethacrylate, gemeint sein können. In einer bevorzugten Ausführungsform ist mit diesem Artikel nur eine einzelne Verbindung gemeint;
- *"mindestens ein", "mindestens eine", "mindestens einer"* zahlenmäßig *"ein oder mehrere".* In einer bevorzugten Ausführungsform ist mit diesem Begriff zahlenmäßig *"ein", "eine", "einer"* gemeint;
- *"enthalten" und "umfassen",* dass neben den genannten Bestandteilen noch weitere vorhanden sein können. Diese Begriffe sind einschließlich gemeint und umfassen daher auch *"bestehen aus". "Bestehen aus"* ist abschließend gemeint und bedeutet, dass keine weiteren Bestandteile vorhanden sein können. In einer bevorzugten Ausführungsform bedeuten die Begriffe *"enthalten" und "umfassen"* den Begriff *"bestehen aus"*;
- *"etwa"* oder *"circa"* vor einem Zahlenwert einen Bereich von ± 5% dieses Wertes, bevorzugt ± 2% dieses Wertes, bevorzugter ± 1% dieses Wertes, besonders bevorzugt ± 0% dieses Wertes (also genau diesen Wert);
- ein durch Zahlen begrenzter Bereich, dass die beiden Eckwerte und jeder Wert innerhalb dieses Bereichs einzeln offenbart sind.

Alle in diesem Text genannten Normen (z.B. DIN-Normen) wurden in der zum Anmeldetag dieser Anmeldung aktuellen Ausgabe verwendet.

Ein erster Gegenstand der Erfindung ist eine Verbindung der allgemeinen Formel (I) 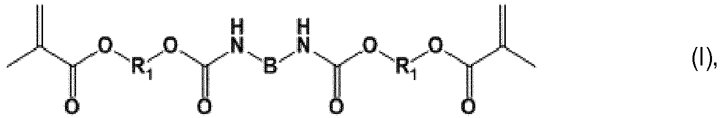 worin
B eine aromatische Kohlenwasserstoffgruppe ist, und
jedes R₁ unabhängig voneinander eine verzweigte oder lineare aliphatische C₁-C₁₅-Alkylengruppe ist.

Ein zweiter Gegenstand ist deren Verwendung zur Herstellung eines Reaktivharzes oder einer Reaktivharzkomponente für bauliche Zwecke. Ein dritter Gegenstand ist deren Verwendung zur Erniedrigung der Viskosität eines Reaktivharzes oder zur Erniedrigung der Auspresskräfte einer Reaktivharzkomponente oder eines Reaktivharz-Systems für bauliche Zwecke. Ein vierter Gegenstand ist deren Verwendung zur Erhöhung der Lastwerte einer ausgehärteten Befestigungsmasse. Ein fünfter Gegenstand ist ein Reaktivharz umfassend die Verbindung der allgemeinen Formel (I), einen Inhibitor, einen Beschleuniger und gegebenenfalls einen Reaktivverdünner. Ein sechster Gegenstand ist eine Reaktivharzkomponente für ein Reaktivharz-System umfassen das Reaktivharz. Ein siebter Gegenstand ist ein Reaktivharz-System mit der Reaktivharzkomponente und einer Härterkomponente, die einen Initiator enthält. Ein achter Gegenstand ist die Verwendung des Reaktivharzes oder des Reaktivharz-Systems für bauliche Zwecke.

Erfindungsgemäß ist die niedrigviskose Urethanmethacrylat-Verbindung eine Verbindung der allgemeinen Formel (I) 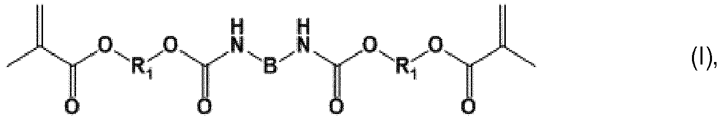 worin B eine aromatische Kohlenwasserstoffgruppe ist, und jedes R₁ unabhängig voneinander eine verzweigte oder lineare aliphatische C₁-C₁₅-Alkylengruppe ist.

Die aromatische Kohlenwasserstoffgruppe ist zweiwertig und hat bevorzugt 6 bis 20 Kohlenstoffatome und weiter bevorzugt 6 bis 14 Kohlenstoffatome. Die aromatische Kohlenwasserstoffgruppe kann substituiert sein, insbesondere durch Alkylreste, worunter Alkylreste mit einem bis vier Kohlenstoffatomen bevorzugt sind.

In einer Ausführungsform enthält die aromatische Kohlenwasserstoffgruppe einen Benzolring, der substituiert sein kann.

In einer alternativen Ausführungsform enthält die aromatische Kohlenwasserstoffgruppe zwei kondensierte Benzolringe oder zwei Benzolringe, die über eine Alkylengruppe, wie eine Methylen oder Ethylengruppe verbrückt sind. Sowohl die Benzolringe als auch die Alkylenbrücke kann substitutiert sein, bevorzugt mit Alkylgruppen.

Die aromatische Kohlenwasserstoffgruppe ist von aromatischen Diisocyanaten abgeleitet, wobei "aromatisches Diisocyanat" bedeutet, dass die beiden Isocyanatgruppen direkt an ein aromatisches Kohlenwasserstoffgerüst gebunden sind.

Geeignete aromatische Kohlenwasserstoffgruppen sind zweiwertige Gruppen, wie sie durch Entfernung der Isocyanatgruppen aus einem aromatischen Diisocyanat erhalten werden, beispielsweise eine zweiwertige Phenylengruppe aus einem Benzoldiisocyanat, eine Methylphenylengruppe aus einem Toluoldiisocyanat (TDI) oder eine Ethylphenylengruppe aus einem Ethylbenzoldiisocyanat, eine zweiwertige Methandiphenylengruppe aus einem Methandiphenyldiisocyanat (MDI) oder eine zweiwertige Naphthylgruppe aus einem Naphthalendiisocyanat (NDI).

Besonders bevorzugt ist B abgeleitet von 1,3-Diisocyanatobenzol, 1,4-Diisocyanatobenzol, 2,4-Diisocyanatotoluol, 2,6-Diisocyanatotoluol, 2,4'-Diphenylmethandiisocyanat, 4,4'-Diphenylmethandiisocyanat oder 1,5-Diisocyanatonaphthalin.

R₁ ist jeweils unabhängig voneinander eine verzweigte oder lineare aliphatische C₁-C₁₅-Alkylengruppe, die substituiert sein kann. R₁ ist von Hydroxyalkylmethacrylaten abgeleitet und umfasst zweiwertige Alkylengruppen, wie sie durch Entfernung der Hydroxylgruppen und der Methyacrylatgruppe erhalten werden.

In einer Ausführungsform ist die Alkylengruppe R₁ zweiwertig.

In einer alternativen Ausführungsform kann sie aber auch drei- oder höherwertig sein, so dass die Verbindung der Formel (I) auch mehr als zwei Methacrylatgruppen aufweisen kann, auch wenn dies nicht direkt aus der Formel (I) ersichtlich ist.

Bevorzugt ist die Alkylengruppe R₁ eine zweiwertige lineare oder verzweigte C₁-C₁₅-Alkylengruppe, bevorzugt C₁-C₆-Alkylengruppe und besonders bevorzugt eine C₁-C₄-Alkylengruppe. Dazu zählen insbesondere die Methylen-, Ethylen-, Propylen-, *i*-Propylen-, *n*-Butylen-, 2-Butylen-, *sec*.-Butylen-, *tert*.-Butylen-, *n*-Pentylen-, 2-Pentylen-, 2-Methylbutylen-, 3-Methylbutylen-, 1,2-Dimethylpropylen-, 1,1-Dimethylpropylen-, 2,2-Dimethylpropylen-, 1-Ethylpropylen-, n-Hexylen-, 2-Hexylen-, 2-Methylpentylen-, 3-Methylpentylen-, 4-Methylpentylen-, 1,2-Dimethylbutylen-, 1,3-Dimethylbutylen-, 2,3-Dimethylbutylen-, 1,1-Dimethylbutylen-, 2,2-Dimethylbutylen-, 3,3-Dimethylbutylen-, 1,1,2-Trimethylpropylen-, 1,2,2-Trimethylpropylen-, 1-Ethylbutylen-, 2-Ethylbutylen-, 1-Ethyl-2-methylpropylen-, *n-*Heptylen-, 2-Heptylen-, 3-Heptylen-, 2-Ethylpentylen-, 1-Propylbutylen- oder die Octylengruppe, worunter die Ethylen-, Propylen- und Isopropylengruppe weiter bevorzugt sind. In einer besonders bevorzugten Ausführungsform der vorliegenden Erfindung sind die beiden R₁-Gruppen identisch und eine Ethylen-, Propylen- oder *i-*Propylengruppe.

Die erfindungsgemäßen niedrigviskosen Urethanmethacrylat-Verbindungen werden durch Umsetzen von zwei Äquivalenten Hydroxyalkylmethacrylat mit mindestens einem Äquivalent Diisocyanat erhalten. Das Diisocyanat und das Hydroxyalkylmethacrylat werden in Anwesenheit eines Katalysators und eines Inhibitors, welcher dazu dient, die gebildete Verbindung zu stabilisieren, zur Reaktion gebracht.

Geeignete Hydroxyalkylmethacrylate sind solche mit Alkylengruppen mit einem bis 15 Kohlenstoffatomen, wobei die Alkylengruppen linear oder verzweigt sein können. Bevorzugt sind Hydroxyalkylmethacrylate mit einem bis 10 Kohlenstoffatomen. Weiter bevorzugt sind Hydroxyalkylmethacrylate mit zwei bis sechs Kohlenstoffatomen, worunter 2-Hydroxyethylmethacrylat, 2-Hydroxypropylmethacrylat (2-HPMA), 3-Hydroxypropylmethacrylat (3-HPMA) und Glycerol-1,3-dimethacrylat besonders bevorzugt sind. Ganz besonders bevorzugt sind 2-Hydroxypropylmethacrylat (2-HPMA) oder 3-Hydroxypropylmethacrylat (3-HPMA).

Bevorzugte aromatische Diisocyanate sind solche mit aromatisch gebundenen Isocyanatgruppen, wie Diisocyanatobenzol, Toluoldiisocyanate (TDI), Diphenylmethandiisocyanate (MDI), Düsocyanatonaphthaline. Diese Verbindungen können sowohl als reine Verbindungen als auch optische Isomere oder Isomerengemische, in unterschiedlicher Zusammensetzung vorliegen, die gegebenenfalls in üblicher Art und Weise getrennt werden können.

Besonders bevorzugte aromatische Diisocyanate sind 1,4-Diisocyanatobenzol, 2,4-Diisocyanatotoluol, 2,6-Diisocyanatotoluol, 2,4'-Diphenylmethandiisocyanat, 4,4'-Diphenylmethandiisocyanat und 1,5-Diisocyanatonaphthalin.

Bevorzugt ist die Verbindung der Formel (I) eine Verbindung der allgemeinen Formel (II) oder (III): 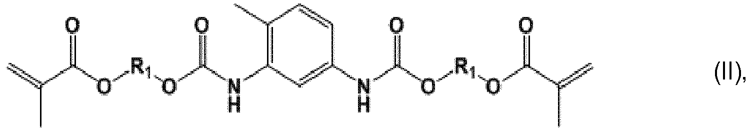 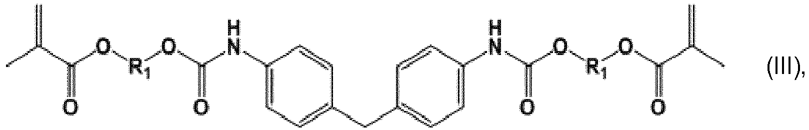 worin jedes R₁ unabhängig voneinander wie oben definiert ist.

Ganz besonders bevorzugt ist die Verbindung der Formel (I) eine Verbindung der Formel (IV) oder (V):

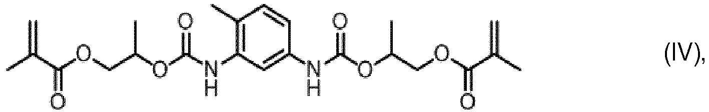

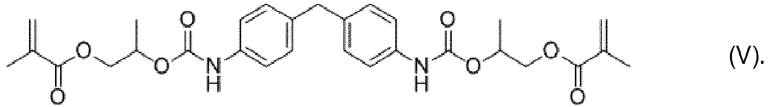

Die in den Formeln (I), (II), (III), (IV) und (V) gezeigten Strukturen sollen die erfindungsgemäßen Verbindungen nur beispielhaft darstellen, da die zu deren Herstellung verwendeten Diisocyanate sowohl als isomerenreine Verbindungen als auch als Gemische der unterschiedlichen Isomere, in jeweils unterschiedlicher Zusammensetzung, d.h. in unterschiedlichen Mengenverhältnissen, eingesetzt werden können. Die gezeigten Strukturen sind daher nicht als einschränkend auszulegen.

Folglich können die erfindungsgemäßen Verbindungen als isomerenreine Verbindungen oder als Isomerengemische, in unterschiedlicher Zusammensetzung vorliegen, die gegebenenfalls in üblicher Art und Weise getrennt werden können. Sowohl die reinen Isomeren als auch die Isomerengemische sind Gegenstand der vorliegenden Erfindung. Gemische mit unterschiedlichen Anteilen an isomeren Verbindungen sind ebenfalls Gegenstand der Erfindung.

Für den Fall, dass bei der Herstellung der erfindungsgemäßen Verbindungen nicht alle Isocyanatgruppen umgesetzt werden oder ein Teil der Isocyanatgruppen vor der Reaktion etwa durch eine Nebenreaktion in andere Gruppen umgewandelt werden, werden Verbindungen erhalten, die entweder als Hauptverbindungen oder als Verunreinigungen in den Reaktionsharz-Masterbatches enthalten sein können. Diese Verbindungen sind, sofern sie für die erfindungsgemäßen Zwecke verwendet werden können auch von der Erfindung umfasst.

Die Verbindungen der Formel (I) werden erfindungsgemäß zur Herstellung eines Reaktivharzes verwendet. Hierdurch kann die Viskosität des so hergestellten Reaktivharzes verringert werden, ohne dass ein hoher Anteil an Reaktivverdünnern erforderlich wird, wie dies bei den handelsüblichen Massen der Fall ist und ohne dass die mit einem hohen Anteil an Reaktivverdünnern einhergehenden Probleme, wie etwa die Verringerung der erreichbaren Lastwerte der ausgehärteten Masse auftreten. Damit kann eine Erniedrigung der Auspresskräfte eines Reaktivharz-Systems, welches die erfindungsgemäßen Verbindungen enthält erreicht werden. Ferner können durch die Verwendung der erfindungsgemäßen Verbindungen die Lastwerte einer ausgehärteten Befestigungsmasse erhöht werden.

Das erfindungsgemäße Reaktivharz enthält eine Verbindung der Formel (I) wie oben beschrieben als Backbone-Harz, einen Inhibitor, einen Beschleuniger und gegebenenfalls einen Reaktivverdünner. Da das Backbone-Harz nach seiner Herstellung typischerweise ohne Isolierung für die Herstellung des Reaktivharzes verwendet wird, sind in der Regel auch noch die neben dem Backbone-Harz im Reaktivharz-Masterbatch enthaltenen weiteren Bestandteile im Reaktivharz vorhanden, wie etwa ein Katalysator.

Der Anteil der Verbindung der allgemeinen Formel (I) in dem erfindungsgemäßen Reaktivharz beträgt von 25 Gew.-% bis 65 Gew.-%, vorzugsweise von 30 Gew.-% bis 45 Gew.-%, besonders bevorzugt von 35 Gew.-% bis 40 Gew.-%, ganz besonders bevorzugt von 33 Gew.-% bis 40 Gew.-% bezogen auf das Gesamtgewicht des Reaktivharzes.

Als Inhibitor sind die für radikalisch polymerisierbare Verbindungen üblicherweise verwendeten stabilen Radikale, wie *N*-Oxyl-Radikale geeignet, wie sie dem Fachmann bekannt sind.

Der Inhibitor kann zum einen dazu dienen, eine unerwünschte radikalische Polymerisation während der Synthese des Backbone-Harzes oder der Lagerung des Reaktivharzes und der Reaktivharzkomponente zu unterdrücken. Er kann auch dazu dienen - gegebenenfalls zusätzlich - die radikalische Polymerisation des Backbone-Harzes nach Zugabe des Initiators zeitlich zu verzögern und dadurch die Verarbeitungszeit des Reaktivharzes oder der Reaktivharzkomponente nach dem Mischen mit dem Härtungsmittel einzustellen.

Als stabile *N*-Oxyl-Radikale können beispielsweise solche verwendet werden, wie sie in der DE 199 56 509 A1 und der DE 195 31 649 A1 beschrieben sind. Derartige stabile Nitroxylradikale sind vom Typ Piperidinyl-*N*-oxyl oder Tetrahydropyrrol-*N*-oxyl oder eine Mischung daraus.

Bevorzugte stabile Nitroxylradikale sind ausgewählt aus der Gruppe bestehend aus 1-Oxyl-2,2,6,6-tetramethylpiperidin, 1-Oxyl-2,2,6,6-tetramethylpiperidin-4-ol (ebenso als TEMPOL bezeichnet), 1-Oxyl-2,2,6,6-tetramethylpiperidin-4-on (ebenso als TEMPON bezeichnet), 1-Oxyl-2,2,6,6-tetramethyl-4-carboxyl-piperidin (ebenso als 4-Carboxy-TEMPO bezeichnet), 1-Oxyl-2,2,5,5-tetramethylpyrrolidin, 1-Oxyl-2,2,5,5-tetramethyl-3-carboxylpyrrolidin (ebenso als 3-Carboxy-PROXYL bezeichnet) und Mischungen aus zwei oder mehreren dieser Verbindungen, wobei 1-Oxyl-2,2,6,6-tetramethylpiperidin-4-ol (TEMPOL) besonders bevorzugt ist. Das TEMPOL ist bevorzugt das in den Beispielen verwendete TEMPOL.

Neben dem Nitroxylradikal vom Typ Piperidinyl-*N*-oxyl oder Tetrahydropyrrol-*N*-oxyl können sowohl zur weiteren Stabilisierung des Reaktivharzes oder der das Reaktivharz enthaltenden Reaktivharzkomponente (A) oder anderer das Reaktivharz enthaltender Zusammensetzungen als auch zur Einstellung der Harzreaktivität ein oder mehrere weitere Inhibitoren vorhanden sein.

Hierfür sind die für radikalisch polymerisierbare Verbindungen üblicherweise verwendeten Inhibitoren geeignet, wie sie dem Fachmann bekannt sind. Bevorzugt sind diese weiteren Inhibitoren unter phenolischen Verbindungen und nicht-phenolischen Verbindungen und/oder Phenothiazinen, ausgewählt.

Als phenolische Inhibitoren, sind Phenole, wie 2-Methoxyphenol, 4-Methoxyphenol, 2,6-Di-tert-butyl-4-methylphenol, 2,4-Di-tert-butylphenol, 2,6-Di-tert-butylphenol, 2,4,6-Trimethylphenol, 2,4,6-Tris(dimethylaminomethyl)phenol, 4,4'-Thio-bis(3-methyl-6-tert-butylphenol), 4,4'-Isopropylidendiphenol, 6,6'-Di-tert-butyl-4,4'-bis(2,6-di-tert-butylphenol), 1,3,5-Trimethyl-2,4,6-tris(3,5-di-tert-butyl-4-hydroxybenzyl)benzol, 2,2'-Methylen-di-p-cresol, Catechole, wie Brenzkatechin, und Catecholderivate, wie Butylbrenzkatechine, wie 4-tert-Butylbrenzkatechin und 4,6-Di-tert-butylbrenzkatechin, Hydrochinone, wie Hydrochinon, 2-Methylhydrochinon, 2-tert-Butylhydrochinon, 2,5-Di-tert-butylhydrochinon, 2,6-Di-tert-butylhydrochinon, 2,6-Dimethylhydrochinon, 2,3,5-Trimethylhydrochinon, Benzochinon, 2,3,5,6-Tetrachloro-1,4-benzochinon, Methylbenzochinon, 2,6-Dimethylbenzochinon, Naphthochinon, oder Gemische von zweien oder mehreren davon, geeignet. Diese Inhibitoren sind oft Bestandteil von kommerziellen radikalisch härtenden Reaktivharzkomponenten.

Als nicht-phenolische Inhibitoren kommen vorzugsweise Phenothiazine, wie Phenothiazin und/oder Derivate oder Kombinationen davon, oder stabile organische Radikale, wie etwa Galvinoxyl- und *N*-oxyl-Radikale, jedoch nicht vom Piperidinyl-*N-*oxyl- oder Tetrahydropyrrol-*N*-oxyl-Typ, in Betracht, wie Aluminium-*N-*nitrosophenylhydroxylamin, Diethylhydroxylamin, Oxime, wie Acetaldoxim, Acetonoxim, Methylethylketoxim, Salicyloxim, Benzoxim, Glyoxime, Dimethylglyoxim, Aceton-O-(benzyloxycarbonyl)oxim und dergleichen.

Ferner können in para-Stellung zur Hydroxylgruppe substituierte Pyrimidinol- oder Pyridinol-Verbindungen, wie sie in der Patentschrift DE 10 2011 077 248 B1 beschrieben sind, als Inhibitoren eingesetzt werden.

Bevorzugt sind die weiteren Inhibitoren ausgewählt aus der Gruppe der Catechole, Catecholderivate, Phenothiazine, tert-Butylcatechol, Tempol oder ein Gemisch von Zwei oder mehr davon. Besonders bevorzugt sind die weiteren Inhibitoren ausgewählt aus der Gruppe der Catechole und Phenothiazine. Ganz besonders bevorzugt sind die in den Beispielen verwendeten weiteren Inhibitoren, bevorzugt etwa in den in den Beispielen angegebenen Mengen.

Die weiteren Inhibitoren können, abhängig von den gewünschten Eigenschaften des Reaktivharzes, entweder alleine oder als Kombination von zweien oder mehreren davon verwendet werden.

Der Inhibitor oder die Inhibitormischung wird zugesetzt in der Fachwelt bekannten üblichen Mengen, bevorzugt in einer Menge von etwa 0,0005 bis etwa 2 Gew.-% (bezogen auf das - letztendlich damit hergestellte - Reaktivharz), stärker bevorzugt von etwa 0,01 bis etwa 1 Gew.-% (bezogen auf das Reaktivharz), noch stärker bevorzugt von etwa 0,05 bis etwa 1 Gew.-% (bezogen auf das Reaktivharz), noch stärker bevorzugt von etwa 0,2 bis etwa 0,5 Gew.-% (bezogen auf das Reaktivharz).

Die Verbindungen der allgemeinen Formel (I), insbesondere bei der Verwendung in Reaktivharzen und Reaktivharzkomponenten für die chemische Befestigung und das bauliche Kleben, werden im Allgemeinen durch Peroxide als Härtungsmittel gehärtet. Die Peroxide werden bevorzugt durch einen Beschleuniger initiiert, damit auch bei niedrigen Anwendungstemperaturen eine Polymerisation stattfindet. Der Beschleuniger wird bereits dem Reaktivharz zugegeben.

Geeignete Beschleuniger, die dem Fachmann bekannt sind, sind beispielsweise Amine, bevorzugt tertiäre Amine und/oder Metallsalze.

Geeignete Amine sind unter folgenden Verbindungen ausgewählt: Dimethylamin, Trimethylamin, Ethylamin, Diethylamin, Triethylamin, n-Propylamin, Di-n-propylamin, Tri-n-propylamin, Isopropylamin, Diisopropylamin, Trüsopropylamin, n-Butylamin, Isobutylamin, tert-Butylamin, Di-n-butylamin, Düsobutylamin, Tri-isobutylamin, Pentylamin, Isopentylamin, Diisopentylamin, Hexylamin, Octylamin, Dodecylamin, Laurylamin, Stearylamin, Aminoethanol, Diethanolamin, Triethanolamin, Aminohexanol, Ethoxyaminoethan, Dimethyl-(2-chloroethyl)amin, 2-Ethylhexylamin, Bis-(2-chloroethyl)amin, 2-Ethylhexylamin, Bis-(2-ethylhexyl)amin, N-Methylstearylamin, Dialkylamine, Ethylendiamin, N,N'-Dimethylethylendiamin, Tetramethylethylendiamin, Diethylentriamin, Permethyldiethylentriamin, Triethylentetramin, Tetraethylenpentamin, 1,2-Diaminopropan, Di-propylentriamin, Tripropylentetramin, 1,4-Diaminobutan, 1,6-Diaminohexan, 4-Amino-1-diethylaminopentan, 2,5-Diamino-2,5-dimethylhexan, Trimethylhexamethylendiamin, N,N-Dimethylaminoethanol, 2-(2-Diethylaminoethoxy)ethanol, Bis-(2-hydroxyethyl)-oleylamin, Tris-[2-(2-hydroxy-ethoxy)-ethyl]amin, 3-Amino-1-propanol, Methyl-(3-aminopropyl)ether, Ethyl-(3-aminopropyl)ether, 1,4-Butandiol-bis(3-aminopropylether), 3-Dimethylamino-1-propanol, 1-Amino-2-propanol, 1-Diethylamino-2-propanol, Diisopropanolamin, Methyl-bis-(2-hydroxypropyl)-amin, Tris-(2-hydroxypropyl)amin, 4-Amino-2-butanol, 2-Amino-2-methylpropanol, 2-Amino-2-methyl-propandiol, 2-Amino-2-hydroxymethylpropandiol, 5-Aiethylamino-2-pentanon, 3-Methylaminopropionsäurenitril, 6-Aminohexansäure, 11-Aminoundecansäure, 6-Aminohexansäureethylester, 11-Aminohexansäureisopropylester, Cyclohexylamin, N-Methylcyclohexylamin, N,N-Dimethylcyclohexylamin, Dicyclohexylamin, N-Ethylcyclohexylamin, N-(2-Hydroxyethyl)-cyclohexylamin, N,N-Bis-(2-hydroxyethyl)-cyclohexylamin, N-(3-Aminopropyl)-cyclohexylamin, Aminomethylcyclohexan, Hexahydrotoluidin, Hexahydrobenzylamin, Anilin, N-Methylanilin, N,N-Dimethylanilin, N,N-Diethylanilin, N,N-Di-propylanilin, iso-Butylanilin, Toluidine, Diphenylamin, Hydroxyethylanilin, Bis-(hydroxyethyl)anilin, Chloranilin, Aminophenole, Aminobenzoesäuren und deren Ester, Benzylamin, Dibenzylamin, Tribenzylamin, Methyldibenzylamin, a-Phenylethylamin, Xylidin, Düsopropylanilin, Dodecylanilin, Aminonaphthalin, N-Methylaminonaphthalin, N,N-Dimethylaminonaphthalin, N,N-Dibenzylnaphthalin, Diaminocyclohexan, 4,4'-Diaminodicyclohexylmethan, Diamino-dimethyl-dicyclohexylmethan, Phenylendiamin, Xylylendiamin, Diaminobiphenyl, Naphthalindiamine, Toluidine, Benzidine, 2,2-Bis-(aminophenyl)-propan, Aminoanisole, Amino-thiophenole, Aminodiphenylether, Aminocresole, Morpholin, N-Methylmorpholin, N-Phenylmorpholin, Hydroxyethylmorpholin, N-Methylpyrrolidin, Pyrrolidin, Piperidin, Hydroxyethylpiperidin, Pyrrole, Pyridine, Chinoline, Indole, Indolenine, Carbazole, Pyrazole, Imidazole, Thiazole, Pyrimidine, Chinoxaline, Aminomorpholin, Dimorpholinethan, [2,2,2]-Diazabicyclooctan und N,N-Dimethyl-p-toluidin.

Als Beschleuniger wird erfindungsgemäß Di-iso-propanol-p-toluidin oder N,N-Bis(2-hydroxyethyl)-m-toluidin eingesetzt.

Bevorzugte Amine sind Anilin-Derivate und N,N-Bisalkylarylamine, wie N,N,-Dimethylanilin, N,N-Diethylanilin, N,N-Dimethyl-p-toluidin, N,N-Bis(hydroxyalkyl)arylamine, N,N-Bis(2-hydroxyethyl)aniline, N,N-Bis(2-hydroxyethyl)toluidin, N,N-Bis(2-hydroxypropyl)anilin, N,N-Bis(2-hydroxypropyl)toluidin, N,N-Bis(3-methacryloyl-2-hydroxypropyl)-p-toluidin, N,N-Dibutoxyhydroxypropyl-p-toluidin und 4,4'-Bis(dimethylamino)diphenylmethan. Besonders bevorzugt ist Di-iso-propanol-p-toluidin.

Polymere Amine, wie solche die durch Polykondensation von N,N-Bis(hydroxylalkyl)anilin mit Dicarbonsäuren oder durch Polyaddition von Ethylenoxid oder anderen Epoxiden und diese Amine erhalten werden, sind ebenso als Beschleuniger geeignet.

Geeignete Metallsalze sind, zum Beispiel Cobaltoctoat oder Cobaltnaphthenoat sowie Vanadium-, Kalium-, Kalzium-, Kupfer-, Mangan- oder Zirkoniumcarboxylate. Weitere geeignete Metallsalze sind die weiter oben beschriebenen Zinn-Katalysatoren.

Sofern ein Beschleuniger verwendet wird, wird er in einer Menge von 0,01 bis 10 Gew.-%, bevorzugt 0,2 bis 5 Gew.-%, bezogen auf das Reaktivharz, eingesetzt.

Das Reaktivharz kann noch einen Reaktivverdünner enthalten, sofern dies erforderlich wird. Dabei kann ein gegebenenfalls bei der Herstellung des Backbone-Harzes eingesetzte Überschuss an hydroxyfunktionalisiertem (Meth)acrylat als Reaktivverdünner fungieren. Daneben, wenn das hydroxyfunktionalisierte (Meth)acrylat in etwa äquimolaren Mengen mit der Isocyanatgruppe eingesetzt wird, oder zusätzlich, falls ein Überschuss an hydroxyfunktionalisiertem (Meth)acrylat verwendet wird, können weitere Reaktivverdünner dem Reaktionsgemisch zugegeben werden, welche strukturell von dem hydroxyfunktionalisierten (Meth)acrylat verschieden sind.

Geeignete Reaktivverdünner sind niederviskose, radikalisch co-polymerisierbare Verbindungen, bevorzugt kennzeichnungsfreie Verbindungen, die zugegeben werden um unter anderem die Viskosität des Urethanmethacrylats bzw. der Vorstufen bei dessen Herstellung anzupassen, falls erforderlich.

Geeignete Reaktivverdünner sind in den Anmeldungen EP 1 935 860 A1 und DE 195 31 649 A1 beschrieben. Vorzugsweise enthält das Reaktivharz (die Harzmischung) als Reaktivverdünner einen (Meth)acrylsäureester, wobei besonders bevorzugt aliphatische oder aromatische C₅-C₁₅-(Meth)acrylate ausgewählt werden. Geeignete Beispiele umfassen: 2-Hydroxypropyl(meth)acrylat, 3-Hydroxypropyl(meth)acrylat, 1,2-Ethandioldi(meth)acrylat, 1,3-Propandioldimethacrylat, 1,3-Butandioldi(meth)acrylat, 1,4-Butandioldi(meth)acrylat, Trimethylolpropantri(meth)acrylat, Phenylethyl(meth)acrylat, Tetrahydrofurfuryl(meth)acrylat, Ethyltriglykol(meth)acrylat, *N,N-*Dimethylaminoethyl(meth)acrylat, N,N-Dimethylaminomethyl(meth)acrylat, N,N-Diethylaminoethyl(meth)acrylat, Acetoacetoxyethyl(meth)acrylat, Isobornyl(meth)acrylat, 2-Ethylhexyl(meth)acrylat, tert-Butylcyclohexyl(meth)acrylat, Benzyl(meth)acrylat, Methyl(meth)acrylat, n-Butyl(meth)acrylat, iso-Butyl(meth)acrylat, 3-Trimethoxysilylpropyl(meth)acrylat, Isodecyl(meth)acrylat, Diethylenglykoldi(meth)acrylat, Triethylenglykoldi(meth)acrylat, Methoxypolyethylenglykolmono(meth)acrylat, Trimethylcyclohexyl(meth)acrylat, 2-Hydroxyethyl(meth)acrylat, Dicyclopentenyloxyethyl(meth)acrylat und/oder Tricyclopentadienyldi(meth)acrylat, Bisphenol-A-(meth)acrylat, Novolakepoxidi(meth)acrylat, Di-[(meth)acryloyl-maleoyl]-tricyclo-5.2.1.0.2.6-decan, 3-(Meth)acryloyl-oxymethyl-tricylo-5.2.1.0.2.6-decan, 3-(Meth)cyclopentadienyl(meth)acrylat, und Decalyl-2-(meth)acrylat; PEG-di(meth)acrylat wie PEG200-di(meth)acrylat, Tetraehtylenglykoldi(meth)acrylat, Solketal(meth)acrylat, Cyclohexyl(meth)acrylat, Phenoxyethyldi(meth)acrylat, 2-Phenoxyethyl(meth)acrylat, Hexandiol-1,6-di(meth)acrylat, 1,2-Butandioldi(meth)acrylat, Methoxyethyl(meth)acrylat, Butyldiglykol(meth)acrylat, tert-Butyl(meth)acrylat und Norbornyl(meth)acrylat. Methacrylate sind gegenüber Acrylaten bevorzugt.

Besonders bevorzugt sind 2- und 3-Hydroxypropylmethacrylat, 1,2-Ethandioldimethacrylat, 1,4-Butandioldimethacrylat, 1,3-Butandioldimethacrylat, Glyceroldimethacrylat, Trimethylolpropantrimethacrylat, Acetoacetoxyethylmethacrylat, Isobornylmethacrylat, Bisphenol-A-dimethacrylat, ethoxylierte Bisphenol-A-methacrylate wie E2BADMA oder E3BADMA, Trimethylcyclohexylmethacrylat, 2-Hydroxyethylmethacrylat, PEG200-dimethacrylat und Norbornylmethacrylat und ganz besonders bevorzugt sind ein Gemisch aus 2- und 3-Hydroxypropylmethacrylat und 1,4-Butandioldimethacrylat oder eine Mischung aus diesen drei Methacrylaten.

Am bevorzugtesten ist ein Gemisch aus 2- und 3-Hydroxypropylmethacrylat. Grundsätzlich können auch andere übliche radikalisch polymerisierbare Verbindungen, allein oder im Gemisch mit den (Meth)acrylsäureestern, als Reaktivverdünner eingesetzt werden, z. B. Methacrylsäure, Styrol, α-Methylstyrol, alkylierte Styrole, wie tert-Butylstyrol, Divinylbenzol und Vinyl- sowie Allylverbindungen, wobei die nicht kennzeichnungspflichtigen Vertreter davon bevorzugt sind. Beispiele für derartige Vinyl- oder Allylverbindungen sind Hydroxybutylvinylether, Ethylenglycoldivinylether, 1,4-Butandioldivinylether, Trimethylolpropandivinylether, Trimethylolpropantrivinylether, Mono-, Di-, Tri-, Tetra- und Polyalkylenglycolvinylether, Mono-, Di-, Tri-, Tetra- und Polyalkylenglycolallylether, Adipinsäuredivinylester, Trimethylolpropandiallylether und Trimethylolpropantriallylether.

Der bzw. die Reaktivverdünner wird bzw. werden in einer Menge bis zu 65 Gew.-%, vorzugsweise bis zu 60 Gew.-%, weiter bevorzugt bis zu 55 Gew.-%, besonders bevorzugt in Mengen unter 50 Gew.-%, bezogen auf das Reaktivharz, zugegeben werden.

Ein beispielhaftes Reaktivharz umfasst eine Verbindung der allgemeinen Formel (I) 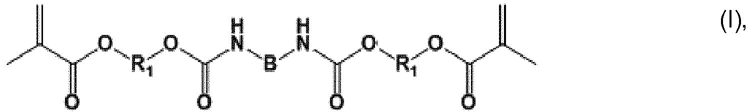 worin B eine aromatische Kohlenwasserstoffgruppe ist, und jedes R₁ unabhängig voneinander eine verzweigte oder lineare aliphatische C₁-C₁₅-Alkylengruppe ist, als Backbone-Harz, ein stabiles Nitroxylradikal als Inhibitor, ein substituiertes Toluidin als Beschleuniger und gegebenenfalls einen Reaktivverdünner.

Ein bevorzugtes Reaktivharz umfasst (a) eine Verbindung der Formel (II) oder (III) 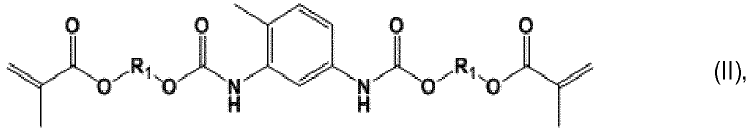 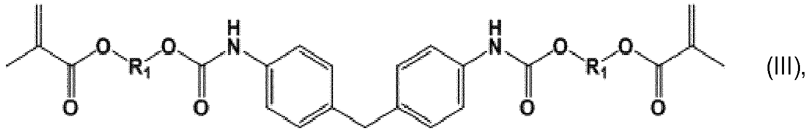 worin jedes R₁ unabhängig voneinander eine verzweigte oder lineare aliphatische C₁-C₁₅-Alkylengruppe ist, als Backbone-Harz, ein stabiles Nitroxylradikal als Inhibitor, ein substituiertes Toluidin als Beschleuniger und gegebenenfalls einen Reaktivverdünner.

Ein weiter bevorzugtes Reaktivharz umfasst eine Verbindung der Formel (IV) oder (V) 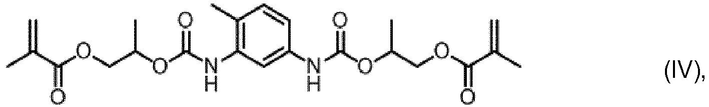 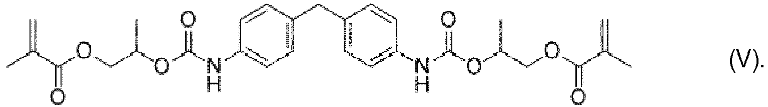 als Backbone-Harz, ein stabiles Nitroxylradikal als Inhibitor, ein substituiertes Toluidin als Beschleuniger und einen Reaktivverdünner.

Ein besonders bevorzugtes Reaktivharz umfasst eine Verbindung der Formel (IV), oder (V) als Backbone-Harz, 4-Hydroxy-2,2,6,6-tetramethyl-piperidinyl-1-oxyl (TEMPOL) als Inhibitor, Di-iso-propanol-p-toluidin als Beschleuniger und eine Mischung aus Hydroxypropylmethacrylat und 1,4-Butandioldimethacrylat (BDDMA) als Reaktivverdünner.

Ein erfindungsgemäßes Reaktivharz weist, aufgrund des niedrigviskosen Backbone-Harzes, besonders niedrige dynamische Viskosität auf, so dass es möglich wird, eine Reaktivharzkomponente für ein Reaktivharz-System herzustellen, welche bei Anwendungstemperaturen unter 10°C, vorzugsweise bei 0°C wesentlich niedrigere Auspresskräfte zeigt als bei den herkömmlichen Systemen, ohne die dafür bisher benötigten hohen Anteile an Reaktivverdünnern.

Ein weiterer Gegenstand der Erfindung ist eine Reaktivharzkomponente, welche das Reaktivharz enthält. Die Reaktivharzkomponente kann neben dem erfindungsgemäßen Reaktivharz anorganische Zuschlagstoffe, wie Füllstoffe und/oder Additive, enthalten. Es sei darauf hingewiesen, dass einige Stoffe sowohl als Füllstoff und, ggf. in modifizierter Form, auch als Additiv verwendet werden können. Beispielsweise dient pyrogene Kieselsäure in ihrer polaren, nicht nachbehandelten Form eher als Füllstoff und in ihrer apolaren, nachbehandelten Form eher als Additiv. In Fällen, in denen genau derselbe Stoff als Füllstoff oder Additiv verwendet werden kann, soll seine Gesamtmenge die hierin festgelegte Obergrenze für Füllstoffe nicht überschreiten.

Zur Herstellung einer Reaktivharzkomponente für bauliche Zwecke, inbesondere die chemische Befestigung können dem erfindungsgemäßen Reaktivharz übliche Füllstoffe und/oder Additive zugegeben werden. Diese Füllstoffe sind typischerweise anorganische Füllstoffe und Additive, wie beispielsweise weiter unten beschrieben.

Der Anteil des Reaktivharzes in der Reaktivharzkomponente beträgt bevorzugt von etwa 10 bis etwa 70 Gew.-%, stärker bevorzugt von etwa 30 bis etwa 50 Gew.-%, bezogen auf die Reaktivharzkomponente. Dementsprechend beträgt der Anteil der Füllstoffe bevorzugt von etwa 90 bis etwa 30 Gew.-%, stärker bevorzugt von etwa 70 bis etwa 50 Gew.-%, bezogen auf die Reaktivharzkomponente.

Als Füllstoffe finden übliche Füllstoffe, vorzugsweise mineralische oder mineralähnliche Füllstoffe, wie Quarz, Glas, Sand, Quarzsand, Quarzmehl, Porzellan, Korund, Keramik, Talkum, Kieselsäure (z.B. pyrogene Kieselsäure, insbesondere polare, nicht nachbehandelte pyrogene Kieselsäure), Silikate, Aluminiumoxide (z.B. Tonerde), Ton, Titandioxid, Kreide, Schwerspat, Feldspat, Basalt, Aluminiumhydroxid, Granit oder Sandstein, polymere Füllstoffe, wie Duroplaste, hydraulisch härtbare Füllstoffe, wie Gips, Branntkalk oder Zement (z.B. Aluminatzement (oft auch als Tonerdezement bezeichnet) oder Portlandzement), Metalle, wie Aluminium, Ruß, ferner Holz, mineralische oder organische Fasern, oder dergleichen, oder Gemische von zwei oder mehr davon Verwendung. Die Füllstoffe können in beliebigen Formen vorliegen, beispielsweise als Pulver oder Mehl, oder als Formkörper, z.B. in Zylinder-, Ring-, Kugel-, Plättchen-, Stäbchen-, Sattel- oder Kristallform, oder ferner in Faserform (fibrilläre Füllstoffe), und die entsprechenden Grundteilchen haben vorzugsweise einen maximalen Durchmesser von etwa 10 mm und einen Minimaldurchmesser von etwa 1 nm. Das heißt, der Durchmesser ist etwa 10 mm oder irgendein Wert von weniger als etwa 10 mm, aber mehr als etwa 1 nm. Bevorzugt ist der maximale Durchmesser ein Durchmesser von etwa 5 mm, bevorzugter von etwa 3 mm, noch bevorzugter von etwa 0,7 mm. Ganz besonders bevorzugt ist ein maximaler Durchmesser von etwa 0,5 mm. Der bevorzugtere Minimaldurchmesser ist etwa 10 nm, noch bevorzugter etwa 50 nm, ganz besonders bevorzugt etwa 100 nm. Durchmesserbereiche, die sich durch Kombination dieser maximalen Durchmesser und Minimaldurchmesser ergeben, sind besonders bevorzugt. Bevorzugt und deutlicher verstärkend wirken sich jedoch die globulären, inerten Stoffe (Kugelform) aus. Auch Core-Shell-Partikel, bevorzugt in Kugelform, können als Füllstoffe verwendet werden.

Bevorzugte Füllstoffe sind ausgewählt aus der Gruppe bestehend aus Zement, Kieselsäure, Quarz, Quarzsand, Quarzmehl, und Mischungen aus zwei oder mehreren davon. Für die Reaktivharzkomponente (A) besonders bevorzugt sind Füllstoffe ausgewählt aus der Gruppe bestehend aus Zement, pyrogener Kieselsäure, insbesondere unbehandelter, polarer pyrogener Kieselsäure, Quarzsand, Quarzmehl, und Mischungen aus zwei oder mehreren davon. Ganz besonders bevorzugt ist für die Reaktivharzkomponente (A) eine Mischung aus Zement (insbesondere Aluminatzement (oft auch als Tonerdezement bezeichnet) oder Portlandzement), pyrogener Kieselsäure und Quarzsand. Für die Härterkomponente (B) ist pyrogene Kieselsäure als alleiniger Füllstoff oder als einer von mehreren Füllstoffen bevorzugt; besonders bevorzugt sind neben der pyrogenen Kieselsäure noch einer oder mehrere weitere Füllstoffe vorhanden.

Als Additive finden übliche Additive Verwendung, also Thixotropiermittel, wie gegebenenfalls organisch oder anorganisch nachbehandelte pyrogene Kieselsäure (falls sie nicht schon als Füllstoff verwendet wird), insbesondere apolar nachbehandelte pyrogene Kieselsäure, Bentonite, Alkyl- und Methylcellulosen, Rhizinusölderivate oder dergleichen, Weichmacher, wie Phthalsäure- oder Sebacinsäureester, weitere Stabilisatoren neben den erfindungsgemäß verwendeten Stabilisatoren und Inhibitoren, Antistatikmittel, Verdickungsmittel, Flexibilisatoren, Rheologiehilfsmittel, Netzmittel, färbende Zusätze, wie Farbstoffe oder insbesondere Pigmente, beispielsweise zum unterschiedlichen Anfärben der Komponenten zur besseren Kontrolle von deren Durchmischung, oder dergleichen, oder Gemische von zwei oder mehr davon. Auch nicht reaktive Verdünnungsmittel (Lösungsmittel) können, vorzugsweise in einer Menge bis zu 30 Gew.-%, bezogen auf die Gesamtmenge der Reaktivharzkomponente enthalten sein, wie Niederalkylketone, z.B. Aceton, Diniederalkyl-niederalkanoylamide, wie Dimethylacetamid, Niederalkylbenzole, wie Xylole oder Toluol, Phthalsäureester oder Paraffine, Wasser oder Glykole. Ferner können Metallfänger in Form von oberflächenmodifizierten pyrogenen Kieselsäuren in der Reaktivharzkomponente enthalten sein. Bevorzugt ist mindestens ein Thixotropiermittel als Additiv vorhanden, besonders bevorzugt eine organisch oder anorganisch nachbehandelte pyrogene Kieselsäure, ganz besonders bevorzugt eine apolar nachbehandelte pyrogene Kieselsäure.

In dieser Hinsicht wird Bezug genommen auf die Anmeldungen WO 02/079341 und WO 02/079293 sowie WO 2011/128061 A1.

Der Anteil der Additive in der Reaktivharzkomponente kann bis zu etwa 5 Gew.-%, bezogen auf die Reaktivharzkomponente, betragen.

Die erfindungsgemäß hergestellten Reaktivharze sind in vielen Bereichen, bei denen sonst üblicherweise ungesättigte Polyesterharze, Vinylesterharze oder Vinylesterurethanharze Verwendung finden, einsetzbar. Sie finden üblicherweise Verwendung als Harzbestandteil in der Reaktivharzkomponente eines Reaktivharz-Systems, wie ein Mehrkomponenten-System, typischerweise ein Zweikomponenten-System aus einer Reaktivharzkomponente (A) und einer Härterkomponente (B). Dieses Mehrkomponenten-System kann in Form eines Patronen-Systems, eines Kartuschen-Systems oder eines Folienbeutel-Systems vorliegen. Bei der bestimmungsgemäßen Verwendung des Systems werden die Komponenten entweder unter Einwirkung mechanischer Kräfte oder durch Gasdruck aus den Patronen, Kartuschen oder Folienbeuteln ausgepresst, miteinander vermischt, vorzugsweise mit Hilfe eines Statikmischers, durch den die Bestandteile hindurchgeführt werden, und appliziert.

Gegenstand der vorliegenden Erfindung ist somit auch ein Reaktivharz-System mit einer eben beschriebenen Reaktivharzkomponente (A) und einer Härterkomponente (B), die einen Initiator für die Urethanmethacrylat-Verbindung enthält.

Der Initiator ist gewöhnlich ein Peroxid. Alle dem Fachmann bekannten Peroxide, die zum Härten von ungesättigten Polyesterharzen und Vinylesterharzen verwendet werden, können eingesetzt werden. Derartige Peroxide umfassen organische und anorganische Peroxide, entweder flüssig oder fest, wobei Wasserstoffperoxid auch verwendet werden kann. Beispiele geeigneter Peroxide sind Peroxycarbonate (der Formel -OC(O)O-), Peroxyester (der Formel -C(O)OO-), Diacylperoxide (der Formel -C(O)OOC(O)-), Dialkylperoxide (der Formel -OO-) und dergleichen. Diese können als Oligomer oder Polymer vorliegen.

Bevorzugt sind die Peroxide aus der Gruppe der organischen Peroxide ausgewählt. Geeignete organische Peroxide sind: tertiäre Alkylhydroperoxide, wie tert-Butylhydroperoxid, und andere Hydroperoxide, wie Cumenhydroperoxid, Peroxyester oder Persäuren, wie tert-Butylperester, Benzoylperoxid, Peracetate und Perbenzoate, Laurylperoxid, einschließlich (Di)peroxyesters, Perether, wie Peroxydiethylether, Perketone, wie Methylethylketoneperoxid. Die als Härter verwendeten organischen Peroxide sind oft tertiäre Perester oder tertiäre Hydroperoxide, d.h. PeroxidVerbindungen mit tertiären Kohlenstoffatomen, die direkt an eine -O-O-acyl- oder-OOH-Gruppe gebunden ist. Aber auch Gemische dieser Peroxide mit anderen Peroxiden können erfindungsgemäß eingesetzt werden. Die Peroxide können auch gemischte Peroxide sein, d.h. Peroxide, die zwei verschiedene Peroxid-tragende Einheiten in einem Molekül aufweisen. Bevorzugt wird zum Härten (Di-benzoyl)peroxid (BPO) verwendet.

Das Reaktivharz-System kann in Form eines Zwei- oder Mehrkomponenten-Systems vorliegen, bei dem die jeweiligen Komponenten räumlich getrennt voneinander vorliegen, so dass eine Reaktion (Aushärtung) der Komponenten erst nach deren Vermischen erfolgt.

Ein Zweikomponenten-Reaktivharz-System umfasst bevorzugt die A Komponente und die B Komponente reaktionsinhibierend getrennt in unterschiedlichen Behältern, beispielsweise einer Mehrkammer-Vorrichtung, wie eine Mehrkammer-Patrone und/oder -Kartusche, aus welchen Behältern die beiden Komponenten durch Einwirkung mechanischer Presskräfte oder unter Einwirkung eines Gasdruckes ausgepresst und vermischt werden. Eine weitere Möglichkeit besteht darin, das Zweikomponenten-Reaktivharzsystem als Zweikomponenten-Kapseln zu konfektionieren, die in das Bohrloch eingeführt werden und durch schlagdrehendes Setzen des Befestigungselements zerstört werden unter gleichzeitiger Durchmischung der beiden Komponenten der Befestigungsmasse. Vorzugsweise wird hierin ein Patronensystem oder ein Injektionssystem eingesetzt, bei dem die beiden Komponenten aus den getrennten Behältern ausgepresst und durch einen statischen Mischer geführt werden, in dem sie homogen vermischt und dann über eine Düse vorzugsweise direkt in das Bohrloch ausgetragen werden.

Bei einer bevorzugten Ausführungsform des erfindungsgemäßen Reaktivharz-Systems ist das Reaktivharz-System ein Zweikomponenten-System und enthält die Reaktivharzkomponente (A) neben dem Backbone-Harz zusätzlich noch eine hydraulisch abbindende oder polykondensierbare anorganische Verbindung, insbesondere Zement, und die Härterkomponente (B) neben dem Initiator für die Polymerisation des Backbone-Harzes noch Wasser. Derartige Hybridmörtelsysteme sind ausführlich in DE 4231161 A1 beschrieben. Dabei enthält die Komponente (A) vorzugsweise als hydraulisch abbindende oder polykondensierbare anorganische Verbindung Zement, beispielsweise Portlandzement oder Tonerdezement, wobei übergangsmetalloxidfreie oder übergangsmetallarme Zemente besonders bevorzugt sind. Als hydraulisch abbindende anorganische Verbindung kann auch Gips als solcher oder in Mischung mit dem Zement eingesetzt werden. Die Komponente (A) kann als polykondensierbare anorganische Verbindung auch silikatische, polykondensierbare Verbindungen, insbesondere lösliches, gelöstes und/oder amorphes Siliziumdioxid enthaltende Stoffe wie z.B. polare, nicht nachbehandelte pyrogene Kieselsäure umfassen.

Das Volumenverhältnis von Komponente A zu Komponente B in einem Zweikomponenten-System ist vorzugsweise 3:1; 5:1 oder 7:1. Besonders bevorzugt ist ein Volumenverhältnis 3:1 oder 5:1.

In einer bevorzugten Ausführungsform enthält die Reaktivharzkomponente (A) also folglich:
- mindestens ein wie oben definiertes Urethan(meth)acrylat, bevorzugt eine Verbindung der Formel (II) oder (III);
- mindestens einen wie oben definierten Inhibitor vom Typ Piperidinyl-*N*-oxyl oder Tetrahydropyrrol-*N*-oxyl, bevorzugt TEMPOL;
- mindestens einen wie oben definierten Beschleuniger, bevorzugt ein Toluidin-Derivat, besonders bevorzugt Di-*iso*-propanol-*p*-toluidin;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Urethan(meth)acrylats, bevorzugt Benzoylperoxid (BPO) oder tert-Butylperoxybenzoat; und
- Wasser.

In einer bevorzugteren Ausführungsform enthält die Reaktivharzkomponente (A):
- mindestens ein wie oben definiertes Urethan(meth)acrylat, bevorzugt eine Verbindung der Formel (II) oder (III);
- mindestens einen wie oben definierten Inhibitor vom Typ Piperidinyl-*N*-oxyl oder Tetrahydropyrrol-*N*-oxyl, bevorzugt TEMPOL;
- mindestens einen Beschleuniger, bevorzugt ein Toluidin-Derivat, besonders bevorzugt Di-iso-propanol-p-toluidin;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Urethan(meth)acrylats, bevorzugt Benzoylperoxid (BPO) oder tert-Butylperoxybenzoat;
- mindestens einen Füllstoff, bevorzugt Quarzsand oder Quarzmehl; und
- Wasser.

In einer noch bevorzugteren Ausführungsform enthält die Reaktivharzkomponente (A):
- mindestens ein wie oben definiertes Urethan(meth)acrylat, bevorzugt eine Verbindung der Formel (II) oder (III);
- mindestens einen wie oben definierten Inhibitor vom Typ Piperidinyl-*N*-oxyl oder Tetrahydropyrrol-*N*-oxyl, bevorzugt TEMPOL;
- mindestens einen Beschleuniger, bevorzugt ein Toluidin-Derivat, besonders bevorzugt Di-*iso*-propanol-*p*-toluidin;
- mindestens einen weiteren Inhibitor, der ausgewählt ist aus der Gruppe besteht aus Catecholen und Phenothiazinen;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Urethan(meth)acrylats, bevorzugt Benzoylperoxid (BPO) oder tert-Butylperoxybenzoat;
- mindestens einen Füllstoff, bevorzugt Quarzsand oder Quarzmehl;
- mindestens ein Thixotropiermittel, bevorzugt pyrogene Kieselsäure; und
- Wasser.

In einer noch bevorzugteren Ausführungsform enthält die Reaktivharzkomponente (A):
- mindestens ein wie oben definiertes Urethan(meth)acrylat, bevorzugt eine Verbindung der Formel (II) oder (III);
- mindestens einen wie oben definierten Inhibitor vom Typ Piperidinyl-*N*-oxyl oder Tetrahydropyrrol-*N*-oxyl, bevorzugt TEMPOL;
- mindestens einen Beschleuniger, bevorzugt ein Toluidin-Derivat, besonders bevorzugt Di-*iso*-propanol-*p*-toluidin;
- mindestens einen weiteren Inhibitor, der ausgewählt ist aus der Gruppe besteht aus Catecholen und Phenothiazinen;
- mindestens eine hydraulisch abbindende oder polykondensierbare anorganische Verbindung, bevorzugt Zement;
- mindestens ein Thixotropiermittel, bevorzugt pyrogene Kieselsäure, und
- mindestens einen weiteren Füllstoff, bevorzugt Quarzsand,
und die Härterkomponente (B) enthält:
- Benzoylperoxid (BPO) odertert-Butylperoxybenzoat als Initiatorfürdie Initiierung der Polymerisation des Urethan(meth)acrylats;
- mindestens einen Füllstoff, bevorzugt Quarzsand oder Quarzmehl;
- mindestens ein Thixotropiermittel, bevorzugt pyrogene Kieselsäure; und
- Wasser.

In einer noch bevorzugteren Ausführungsform enthält die Reaktivharzkomponente (A):
- mindestens ein wie oben definiertes Urethan(meth)acrylat, bevorzugt eine Verbindung der Formel (IV) oder (V);
- TEMPOL;
- Di-*iso*-propanol-*p*-toluidin;
- mindestens einen weiteren Inhibitor, der ausgewählt ist aus der Gruppe besteht aus Catecholen und Phenothiazinen;
- Zement;
- pyrogene Kieselsäure; und
- Quarzsand,
und die Härterkomponente (B) enthält:
- mindestens einen Initiator für die Initiierung der Polymerisation des Urethan(meth)acrylats;
- pyrogene Kieselsäure;
- Quarzsand oder Quarzmehl und
- Wasser.

In jeder dieser Ausführungsformen enthält in einer bevorzugten Ausführungsform die Reaktivharzkomponente (A) zusätzlich noch mindestens einen Reaktivverdünner. Bevorzugt ist dieser Reaktivverdünner ein Monomer oder eine Mischung aus mehreren Monomeren des Backbone-Harzes.

Die Reaktivharzkomponenten (A) und die Härterkomponenten (B) in jeder dieser Ausführungsformen sind beliebig miteinander kombinierbar.

Ein solches Reaktivharz-System findet vor allem im Baubereich Verwendung (bauliche Zwecke), beispielsweise bei der Erstellung und Instandhaltung bzw. -setzung von Bauteilen und Bauwerken, etwa aus Beton, als Polymerbeton, als Beschichtungsmasse auf Kunstharzbasis oder als kalthärtende Straßenmarkierung, zur Verstärkung von Bauteilen und Bauwerken, beispielsweise Wände, Decken oder Böden, die Befestigung von Bauteilen, wie Platten oder Blöcken, z. B. aus Stein, Glas oder Kunststoff, an Bauteilen oder Bauwerken, etwa durch Kleben (bauliches Kleben). Besonders eignet sie sich zur chemischen Befestigung. Ganz besonders eignet sie sich zur (stoff- und/oder formschlüssigen) chemischen Befestigung von Verankerungsmitteln, wie Ankerstangen, Bolzen, Bewehrungseisen, Schrauben oder dergleichen in Aussparungen, wie Bohrlöchern, insbesondere in Bohrlöchern in verschiedenen Untergründen, insbesondere mineralischen Untergründen, wie solche auf der Grundlage von Beton, Porenbeton, Ziegelwerk, Kalksandstein, Sandstein, Naturstein, Glas und dergleichen, und metallischen Untergründen, wie solche aus Stahl. In einer Ausführungsform ist der Untergrund des Bohrlochs Beton, und das Verankerungsmittel besteht aus Stahl oder Eisen. In einer weiteren Ausführungsform ist der Untergrund des Bohrlochs Stahl, und das Verankerungsmittel besteht aus Stahl oder Eisen. Hierzu werden die Komponenten in das Bohrloch injiziert, wonach die zu befestigenden Einrichtungen, wie Ankergewindestangen und dergleichen, in das mit dem aushärtenden Reaktivharz beschickte Bohrloch eingebracht und entsprechend justiert werden.

Die folgenden Beispiele dienen der weiteren Erläuterung der Erfindung.

### BEISPIELE

Zunächst wurden Reaktivharze, Reaktivharzkomponenten und Zweikomponenten-Reaktivharz-Systeme, die jeweils die erfindungsgemäße Verbindung (IV) als Backbone-Harz enthalten, hergestellt. Die dynamische Viskosität der Reaktivharze und der Reaktivharzkomponenten, die Auspresskräfte der Zweikomponenten-Reaktivharz-Systeme und die Verbundspannungen der ausgehärteten Befestigungsmassen wurden ermittelt.

### Erfindungsgemäße Verbindung (IV)

### A1. Herstellung des Reaktivharz-Masterbatches A1 mit Verbindung (IV)

In einem 2 Liter Laborglasreaktor mit Innenthermometer und Rührwelle wurden 1542 g Hydroxypropylmethacrylat vorgelegt und mit 0,24 g Phenothiazin (D Prills; Allessa Chemie), 0,60 g 4-Hydroxy-2,2,6,6-tetramethyl-piperidinyl-1-oxyl (TEMPOL; Evonik Degussa GmbH) und 0,40 g Dioctylzinndilaurat (TIB KAT^{®} 216; TIB Chemicals) versetzt. Der Ansatz wurde auf 80°C erwärmt. Anschließend wurden 500 g Toluol-2,4-diisocyanat (TDI; TCI Deutschland GmbH) unter Rühren beim 200 U/min über 45 Minuten zugegeben. Danach wurde weiter 180 Minuten bei 80°C gerührt.

Hierdurch wurde der Reaktivharz-Masterbatch A1 erhalten, der 65 Gew.-% der Verbindung (IV) als Backbone-Harz und 35 Gew.-% Hydroxypropylmethacrylat, bezogen auf das Gesamtgewicht des Reaktivharz-Masterbatches, enthält.

Die Verbindung (IV) weist die folgende Struktur auf:

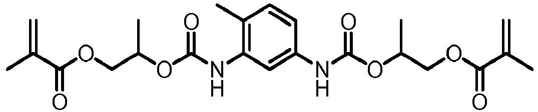

Aus dem Reaktivharz-Masterbatch A1 wurde ein Reaktivharz (A2.1) mit 33 Gew.-% und ein Reaktivharz (A2.2) mit 41 Gew.-% an Verbindung (IV) als Backbone-Harz hergestellt.

### A2.1 Herstellung des Reaktivharzes A2.1 mit 33 Gew.-% Verbindung (IV)

301 g Reaktivharz-Masterbatch A1 wurden mit 90 g Hydroxypropylmethacrylat und 196 g 1,4-Butandioldimethacrylat (BDDMA; Evonik AG) gemischt. Zu dieser Mischung wurden 2,75 g 4-Hydroxy-2,2,6,6-tetramethyl-piperidinyl-1-oxyl (TEMPOL; Evonik Degussa GmbH) und 10,5 g Di-iso-propanol-p-toluidin (BASF SE) gegeben.

Hierdurch wurde das Reaktivharz A2.1 mit 33 Gew.-% Anteil an Verbindung (IV) als Backbone-Harz erhalten.

### A2.2 Herstellung des Reaktivharzes A2.2 mit 41 Gew.-% Verbindung (IV)

376 g Reaktivharz-Masterbatch A1 wurden mit 39 g Hydroxypropylmethacrylat und 171 g 1,4-Butandioldimethacrylat (BDDMA; Evonik AG) gemischt. Zu dieser Mischung wurden 2,75 g 4-Hydroxy-2,2,6,6-tetramethyl-piperidinyl-1-oxyl (TEMPOL; Evonik Degussa GmbH) und 10,5 g Di-iso-propanol-p-toluidin (BASF SE) gegeben.

Hierdurch wurde das Reaktivharz A2.2 mit 41 Gew.-% Anteil an Verbindung (IV) als Backbone-Harz erhalten.

### A3. Herstellung der Reaktivharzkomponenten A3.1 und A3.2

354 g Reaktivharz A2.1 bzw. A2.2 wurden mit 185 g Secar^{®} 80 (Kerneos Inc.), 27 g Cab-O-Sil^{®} TS-720 (Cabot Corporation) und 335 g Quarzsand F32 (Quarzwerke GmbH) mit einem PC Laborsystem Dissolver vom Typ LDV 0.3-1 für 8 Minuten bei 3500 U/min unter Vakuum (Druck≤100 mbar) mit einer 55 mm Dissolverscheibe und einem Randabstreifer vermischt.

Hierdurch wurden die Reaktivharzkomponenten A3.1 und A3.2 erhalten.

Daraus wurden Reaktivharz-Systeme als zweikomponentige Systeme hergestellt.

### A4. Herstellung der Zweikomponenten-Reaktivharz-Systeme A4.1 und A4.2

Zur Herstellung der Zweikomponenten-Reaktivharz-Systeme A4.1 und A4.2 wurden die Reaktivharzkomponenten A3.1 und A3.2 (Komponente (A)) und jeweils die Härterkomponente (Komponente (B)) des kommerziell erhältlichen Produkts HIT-HY 110 (Hilti Aktiengesellschaft; Chargennummer: 1610264) in Plastikkartuschen (Firma Ritter GmbH; Volumenverhältnis A:B = 3:1) mit den Innendurchmessern 47 mm (Komponente (A)) bzw. 28 mm (Komponente (B)) gefüllt.

Hierdurch wurden die Zweikomponenten-Reaktivharz-Systeme A4.1 (mit 33 Gew.-% Anteil an Verbindung (IV) im Reaktivharz) und A4.2 (mit 41 Gew.-% Anteil an Verbindung (IV) im Reaktivharz) erhalten.

Um einen höheren Anteil an Verbindung (IV) in ein Reaktivharz einzubringen, wurde ein weiterer Reaktivharz-Masterbatch (B1) mit einem hohen Anteil von 80 Gew.-% an Verbindung (IV) hergestellt.

### B1. Herstellung des Reaktivharz-Masterbatches B1 mit Verbindung (IV)

In einem 2 Liter Laborglasreaktor mit Innenthermometer und Rührwelle wurden 1396 g Hydroxypropylmethacrylat vorgelegt und mit 0,29 g Phenothiazin (D Prills; Allessa Chemie), 0,70 g 4-Hydroxy-2,2,6,6-tetramethyl-piperidinyl-1-oxyl (TEMPOL; Evonik Degussa GmbH) und 0,49 g Dioctylzinndilaurat (TIB KAT 216; TIB Chemicals) versetzt. Der Ansatz wurde auf 80°C erwärmt. Anschließend wurden 602 g Toluol-2,4-diisocyanat (TCI Deutschland GmbH) unter Rühren beim 200 U/min über 45 Minuten zugegeben. Danach wurden weitere 180 Minuten bei 80°C gerührt.

Hierdurch wurde der Reaktivharz-Masterbatch B1 erhalten, der 80 Gew.-% Verbindung (IV) als Backbone-Harz und 20 Gew.-% Hydroxypropylmethacrylat, bezogen auf das Gesamtgewicht des Reaktivharz-Masterbatches, enthält.

Aus dem Reaktivharz-Masterbatch B1 wurden ebenfalls Reaktivharze mit unterschiedlichem Anteil an Verbindung (IV) als Backbone-Harz hergestellt.

### B2.1 Herstellung des Reaktivharzes B2.1 mit 37 Gew.-% Verbindung (IV)

186 g Reaktivharz-Masterbatch aus B1 wurden mit 43 g Hydroxypropylmethacrylat und 160 g 1,4-Butandioldimethacrylat (BDDMA; Evonik AG) gemischt. Zu dieser Mischung wurden 1,08 g Brenzcatechin (Hersteller Solvay Catechol Flakes) und 0,36 g 4-tert-Butylbrenzcatechin und 9,2 g Di-iso-propanol-p-toluidin (BASF SE) gegeben.

Hierdurch wurde das Reaktivharz B2.1 mit 37 Gew.-% Anteil an Verbindung (IV) als Backbone-Harz erhalten.

### B2.2 Herstellung des Reaktivharzes B2.2 mit 40 Gew.-% Verbindung (IV)

200 g Reaktivharz-Masterbatch B1 wurden mit 37 g Hydroxypropylmethacrylat und 153 g 1,4-Butandioldimethacrylat (BDDMA; Evonik AG) gemischt. Zu dieser Mischung wurden 1,08 g Brenzcatechin (Solvay Catechol Flakes) und 0,36 g 4-tert-Butylbrenzcatechin und 9,2 g Di-iso-propanol-p-toluidin (Hersteller BASF SE) gegeben.

Hierdurch wurde das Reaktivharz B2.2 mit 40 Gew.-% Anteil an Verbindung (IV) als Backbone-Harz erhalten.

### B2.3 Herstellung des Reaktivharzes B2.3 mit 45 Gew.-% Verbindung (IV)

225 g Reaktivharz-Masterbatch B1 wurden mit 26 g Hydroxypropylmethacrylat und 140 g 1,4-Butandioldimethacrylat (BDDMA; Evonik AG) gemischt. Zu dieser Mischung wurden 1,08 g Brenzcatechin (Solvay Catechol Flakes) und 0,36 g 4-tert-Butylbrenzcatechin und 9,2 g Di-iso-propanol-p-toluidin (BASF SE) gegeben.

Hierdurch wurde das Reaktivharz B2.3 mit 45 Gew.-% Anteil an Verbindung (IV) als Backbone-Harz erhalten.

### B2.4 Herstellung des Reaktivharzes B2.4 mit 50 Gew.-% Verbindung (IV)

250 g Reaktivharz-Masterbatch B1 wurden mit 13 g Hydroxypropylmethacrylat und 127 g 1,4-Butandioldimethacrylat (BDDMA; Evonik AG) gemischt. Zu dieser Mischung wurden 1,08 g Brenzcatechin (Solvay Catechol Flakes) und 0,36 g 4-tert-Butylbrenzcatechin und 9,2 g Di-iso-propanol-p-toluidin (BASF SE) gegeben.

Hierdurch wurde das Reaktivharz B2.4 mit 50 Gew.-% Anteil an Verbindung (IV) als Backbone-Harz erhalten.

### B3. Herstellung der Reaktivharzkomponenten B3.1, B3.2, B3.3 und B3.4

Jeweils 311 g Reaktivharz B2.1, B2.2, B2.3 und B2.4 wurden mit 167 g Secar^{®} 80 (Kerneos Inc.), 9 g Cab-O-Sil^{®} TS-720 (Cabot Corporation), 16 g Aerosil^{®} R812 (Evonik Industries AG) und 398 g Quarzsand F32 (Quarzwerke GmbH) im Dissolver unter Vakuum vermischt. Das Mischen erfolgte mit einem PC Laborsystem Dissolver vom Typ LDV 0.3-1, wie unter Punkt A3 beschrieben.

Hierdurch wurden die Reaktivharzkomponenten B3.1, B3.2, B3.3 und B3.4 erhalten, die die Verbindung (IV) als Backbone-Harz enthielten.

Daraus wurden Reaktivharz-Systeme als zweikomponentige Systeme hergestellt.

### B4. Herstellung der Zweikomponenten-Reaktivharz-Systeme B4.1 bis B4.4

Zur Herstellung der Zweikomponenten-Reaktivharz-Systeme B4.1, B4.2, B4.3 und B4.4 wurden die Reaktivharzkomponenten B3.1, B3.2, B3.3 und B3.4 (Komponente (A)) und jeweils die Härterkomponente (Komponente (B)) des kommerziell erhältlichen Produkts HIT HY-200 (Hilti Aktiengesellschaft; Chargennummer: 8104965) in Plastikkartuschen (Firma Ritter GmbH; Volumenverhältnis A:B = 5:1) mit den Innendurchmessern 32,5 mm (Komponente (A)) bzw. 14 mm (Komponente (B)) gefüllt.

Hierdurch wurden die Zweikomponenten-Reaktivharz-Systeme B4.1 (mit 37 Gew.-% Anteil an Verbindung (IV) im Reaktivharz), B4.2 (mit 40 Gew.-% Anteil an Verbindung (IV) im Reaktivharz), B4.3 (mit 45 Gew.-% Anteil an Verbindung (IV) im Reaktivharz) und B4.4 (mit 50 Gew.-% Anteil an Verbindung (IV) im Reaktivharz) erhalten.

### Vergleichsbeispiele C und D

Zum Vergleich wurden Reaktivharz-Masterbatches, Reaktivharze und Reaktivharzkomponenten mit den Vergleichsverbindungen 1 und 2 wie folgt hergestellt.

### C1. Herstellung des Vergleichsreaktivharz-Masterbatches C1 mit Vergleichsverbindung 1

Der Vergleichsreaktivharz-Masterbatch C1 mit 65 Gew.-% an Vergleichsverbindung 1 als Backbone-Harz und 35 Gew.-% Hydroxypropylmethacrylat wurde gemäß dem Verfahren in EP 0 713 015 A1, die hiermit als Referenz eingeführt und auf deren gesamte Offenbarung verwiesen wird, hergestellt.

Das Produkt (Vergleichsverbindung 1) hat eine Oligomerverteilung, wobei das Oligomer mit einer Wiederholungseinheit die folgende Struktur hat:

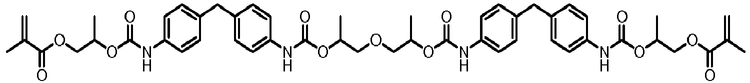

### C2.1 Herstellung des Vergleichsreaktivharzes C2.1 mit 33 Gew.-% Vergleichsverbindung 1

9,2 g 4-Hydroxy-2,2,6,6-tetramethyl-piperidinyl-1-oxyl (TEMPOL) und 35,0 g Di-iso-propanol-p-toluidin (BASF SE) wurden zu einer Mischung aus 1004 g Reaktivharz-Masterbatch C1, 300 g Hydroxypropylmethacrylat und 652 g 1,4-Butandioldimethacrylat (BDDMA; Evonik AG) gegeben.

Hierdurch wurde das Vergleichsreaktivharz C2.1 mit 33 Gew.-% Vergleichsverbindung 1 als Backbone-Harz erhalten.

### C2.2 Herstellung des Vergleichsreaktivharzes 1 mit 37 Gew.-% Vergleichsverbindung 1

229 g Vergleichsreaktivharz-Masterbatch C1 wurden mit 160 g 1,4-Butandioldimethacrylat (BDDMA; Evonik AG) gemischt. Zu dieser Mischung wurden 1,08 g Brenzcatechin (Solvay, Catechol Flakes) und 0,36 g 4-tert-Butylbrenzcatechin (tBBK, CFS EUROPE S.p.A. (Borregaard Italia S.p.A.)) und 9,2 g Di-iso-propanol-p-toluidin (BASF SE) gegeben.

Hierdurch wurde das Vergleichsreaktivharz C2.2 mit 37 Gew.-% Vergleichsverbindung 1 als Backbone-Harz erhalten.

### C2.3 Herstellung des Vergleichsreaktivharzes C2.3 mit 41 Gew.-% Vergleichsverbindung 1

2,8 g 4-Hydroxy-2,2,6,6-tetramethyl-piperidinyl-1-oxyl (TEMPOL; Evonik Degussa GmbH) und 10,5 g Di-iso-propanol-p-toluidin (BASF SE) wurden zu einer Mischung aus 377 g Vergleichsreaktivharz-Masterbatch C1, 39 g Hydroxypropylmethacrylat und 171 g 1,4-Butandioldimethacrylat gegeben.

Hierdurch wurde das Vergleichsreaktivharz C2.3 mit 41 Gew.-% Vergleichsverbindung 1 als Backbone-Harz erhalten.

### C3. Herstellung der Vergleichsreaktivharzkomponenten C3.1 bis C3.3

Jeweils 354 g Vergleichsreaktivharz C2.1 bzw. C2.3 wurden mit 185 g Secar^{®} 80 (Kerneos Inc.), 27 g Cab-O-Sil^{®} TS-720 (Cabot Corporation) und 335 g Quarzsand F32 (Quarzwerke GmbH) im Dissolver unter Vakuum vermischt. Das Mischen erfolgte mit einem PC Laborsystem Dissolver vom Typ LDV 0.3-1, wie unter Punkt A3 beschrieben.

Hierdurch wurden die Vergleichsreaktivharzkomponenten C3.1 mit 33 Gew.-% Vergleichsverbindung 1 im Reaktivharz (aus C2.1) und C3.2 mit 41 Gew.-% Vergleichsverbindung 1 im Reaktivharz (aus C2.3) erhalten.

311 g Vergleichsreaktivharz C2.2 wurden mit 167 g Secar^{®} 80 (Kerneos Inc.), 9 g Cab-O-Sil^{®} TS-720 (Cabot Corporation), 16 g Aerosil^{®} R812 (Evonik Industries AG) und 398 g Quarzsand F32 (Quarzwerke GmbH) im Dissolver unter Vakuum vermischt. Das Mischen erfolgte mit einem PC Laborsystem Dissolver vom Typ LDV 0.3-1, wie unter Punkt A3 beschrieben.

Hierdurch wurde die Vergleichsreaktivharzkomponenten C3.3 mit 37 Gew.-% Vergleichsverbindung im Reaktivharz (aus C2.2) erhalten.

### C4. Herstellung der Vergleichszweikomponenten-Reaktivharz-Systeme C4.1 bis C4.3

Zur Herstellung der Vergleichszweikomponenten-Reaktivharz-Systeme C4.1 und C4.2 wurden die Reaktivharzkomponenten C3.1 und C3.2 (Komponente (A)) und jeweils die Härterkomponente (Komponente (B)) des kommerziell erhältlichen Produkts HIT-HY 110 (Hilti Aktiengesellschaft; Chargennummer: 1610264) in Plastikkartuschen (Firma Ritter GmbH; Volumenverhältnis A:B = 3:1) mit den Innendurchmessern 47 mm (Komponente (A)) bzw. 28 mm (Komponente (B)) gefüllt.

Hierdurch wurden die Zweikomponenten-Vergleichsreaktivharz-Systeme C4.1 mit 33 Gew.-% Vergleichsverbindung 1 im Reaktivharz (aus C3.1) und C4.2 mit 41 Gew.-% Vergleichsverbindung 1 im Reaktivharz (aus C3.2) erhalten.

Zur Herstellung des Vergleichszweikomponenten-Reaktivharz-Systems C4.3 wurden die Reaktivharzkomponenten C3.3 (Komponente (A)) und jeweils die Härterkomponente (Komponente (B)) des kommerziell erhältlichen Produkts HIT-HY 200 (Hilti Aktiengesellschaft; Chargennummer: 8104965) in Plastikkartuschen (Firma Ritter GmbH; Volumenverhältnis A:B = 5:1) mit den Innendurchmessern 32,5 mm (Komponente (A)) bzw. 14 mm (Komponente (B)) gefüllt.

Hierdurch wurde das Zweikomponenten-Vergleichsreaktivharz-System C4.3 mit 37 Gew.-% Vergleichsverbindung 1 im Reaktivharz (aus C3b) erhalten.

### D1. Herstellung des Vergleichsreaktivharz-Masterbatches D1 mit Vergleichsverbindung 2

Der Vergleichsreaktivharz-Masterbatch D1 mit jeweils 65 Gew.-% Vergleichsverbindung 2 als Backbone-Harz und 35 Gew.-% Hydroxypropylmethacrylat, jeweils bezogen auf das Gesamtgewicht des Reaktivharz-Masterbatches, wurden gemäß dem Verfahren in EP 0 713 015 A1, die hiermit als Referenz eingeführt und auf deren gesamte Offenbarung verwiesen wird, hergestellt.

Die Vergleichsverbindung 2 hat folgende Struktur:

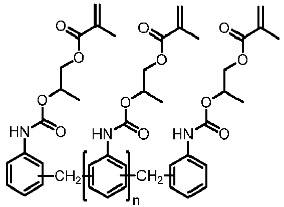

Daraus wurden Vergleichsreaktivharze mit unterschiedlich hohem Anteil an Vergleichsverbindung 2 hergestellt.

### D2. Herstellung des Vergleichsreaktivharzes D2

4,6 g 4-Hydroxy-2,2,6,6-tetramethyl-piperidinyl-1-oxyl (TEMPOL; Evonik Degussa GmbH) und 17,5 g Di-iso-propanol-p-toluidin (BASF SE) wurden zu einer Mischung aus 502 g Vergleichsreaktivharz-Masterbatch D1, 150 g Hydroxypropylmethacrylat und 326 g 1,4-Butandioldimethacrylat (1,4-BDDMA; Evonik Degussa GmbH) gegeben.

Hierdurch wurde das Vergleichsreaktivharz D2 mit Vergleichsverbindung 2 als Backbone-Harz erhalten.

### D3. Herstellung der Vergleichsreaktivharzkomponente D3

354 g Vergleichsreaktivharz D2 wurden mit 185 g Secar^{®} 80 (Kerneos Inc.), 27 g Cab-O-Sil^{®} TS-720 (Cabot Corporation) und 335 g Quarzsand F32 (Quarzwerke GmbH) im Dissolver unter Vakuum vermischt. Das Mischen erfolgte mit einem PC Laborsystem Dissolver vom Typ LDV 0.3-1, wie unter Punkt A3 beschrieben.

Hierdurch wurde die Vergleichsreaktivharzkomponente D3 mit der Vergleichsverbindung 2 als Backbone-Harz erhalten.

### D4. Herstellung des Veraleichszweikomponenten-Reaktivharz-Systems D4

Zur Herstellung des Vergleichszweikomponenten-Reaktivharz-Systems D4 wurde die Vergleichsreaktivharzkomponente D3 (Komponente (A)) und die Härterkomponente (Komponente (B)) des kommerziell erhältlichen Produkts HIT-HY 110 (Hilti Aktiengesellschaft; Chargennummer: 1610264) in eine Plastikkartusche (Firma Ritter GmbH; Volumenverhältnis A:B = 3:1) mit den Innendurchmessern 47 mm (Komponente (A)) bzw. 28 mm (Komponente (B)) gefüllt.

Hierdurch wurde das Vergleichszweikomponenten-Reaktivharz-System D4 erhalten.

Um den Einfluss der erfindungsgemäßen Verbindung (IV) auf die Viskosität eines diese enthaltenden Reaktivharz-Masterbatches, eines Reaktivharzes und einer Reaktivharzkomponente und den Einfluss auf die Verbundspannungen einer erhärteten Befestigungsmasse zu zeigen, wurden die Viskositäten der erfindungsgemäßen Reaktivharz-Masterbatches, Reaktivharze, Reaktivharzkomponenten, die Auspresskräfte von Zweikomponenten-Reaktivharz-Systemen sowie die Verbundspannungen von ausgehärteten Befestigungsmassen gemessen und jeweils mit den Vergleichsformulierungen verglichen.

### Messung der dynamischen Viskosität des Reaktivharz-Masterbatches A1 und der Vergleichsreaktivharz-Masterbatches C1 und D1

Die Messung der dynamischen Viskosität des Reaktivharz-Masterbatches A1 und der Vergleichsreaktivharz-Masterbatches C1 und D1 (Tabelle 1) erfolgte mit einem Kegel-Platte Messsystem nach DIN 53019. Der Durchmesser des Kegels betrug 20mm und der Öffnungswinkel war 1 °. Es wurde bei einer konstanten Schergeschwindigkeit von 100/s bei der jeweiligen Temperatur gemessen (0, 5, 10, 15, 20, 30 und 40°C). Die Messdauer betrug 120s und jede Sekunde wurde ein Messpunkt generiert. Zur Erreichung der Schergeschwindigkeit bei der jeweiligen Temperatur wurde eine Rampe von 0-100/s mit einer Dauer von 30s vorgeschaltet. Da es sich um newtonschen Flüssigkeiten handelt, wurde über den Messabschnitt mit konstanter Schergeschwindigkeit von 100/s eine lineare Auswertung über den Messabschnitt vorgenommen und die Viskosität bestimmt. Es wurden jeweils drei Messungen gemacht, wobei in Tabelle 1 jeweils die Mittelwerte angegeben sind.

### Messung der dynamischen Viskosität der Reaktivharzkomponenten A3.1, B3.1, B3.2, B3.3 und B3.4 sowie der Vergleichsreaktivharzkomponenten C3.1, C3.3 und D3

Die Messung der dynamischen Viskosität der erfindungsgemäßen Reaktivharzkomponente A3 und der Vergleichsreaktivharzkomponenten C3 und D3 (Tabelle 2) sowie der Reaktivharzkomponenten B3.1, B3.2, B3.3 und B3.4 und der Vergleichsreaktivharzkomponenten C3.3 (Tabelle 3) erfolgte mit einem Platte-Platte Messsystem nach DIN 53019. Der Durchmesser der Platte betrug 20mm und der Spaltabstand betrug 3mm. Um ein Austreten der Probe aus dem Spalt zu verhindern, wurde ein Begrenzungsring aus Teflon verwendet, der einen Abstand von 1 mm von der oberen Platte hatte. Die Messtemperatur betrug 25°C. Die Methode bestand aus drei Abschnitten: 1. Niedrige Scherung, 2. Hohe Scherung, 3. Niedrige Scherung. Beim 1. Abschnitt wurde 3 Minuten bei 0,5/s geschert. Im 2. Abschnitt wurde in 8 Stufen zu je 15 Sekunden logarithmisch die Schergeschwindigkeit von 0,8/s auf 100/s gesteigert. Die einzelnen Stufen dabei waren: 0,8/s; 1,724/s; 3,713/s; 8/s; 17,24/s; 37,13/s; 80/s; 100/s. Der 3. Abschnitt war die Wiederholung des 1 Abschnitts. Am Ende eines jeden Abschnittes wurden die Viskositäten ausgelesen. In Tabelle 2 wird der Wert des zweiten Abschnittes bei 100/s angegeben. Es wurden jeweils drei Messungen durchgeführt, wobei die in Tabelle 2 angegebenen Werte der Mittelwert der drei Messungen ist.

Zunächst wurde die dynamische Viskosität des Reaktivharz-Masterbatches A1 mit den Vergleichsreaktivharz-Masterbatches C1 und D1 bei unterschiedlichen Temperaturen verglichen (Tabelle 1). Die Reaktivharz-Masterbatches enthielten jeweils 65 Gew.-% Backbone-Harz und 35 Gew.-% Hydroxypropylmethacrylat.

**Tabelle 1: Ergebnisse der Messungen der dynamischen Viskosität des Reaktivharz-Masterbatches A1 und der Vergleichsreaktivharz-Masterbatches C1 und D1 bei unterschiedlichen Temperaturen**

| | **Reaktivharz-Masterbatch A1** | **Vergleichsreaktivharz-Masterbatch C1** | **Vergleichsreaktivharz-Masterbatch D1** |
|---|---|---|---|
| **T [°C]** | **Viskosität [mPa·s]** | | |
| 0 | 45.390 | 188.000 | 281.200 |
| 5 | 18.970 | 81.520 | 110.500 |
| 10 | 8.325 | 37.050 | 45.020 |
| 15 | 3.815 | 17.280 | 19.470 |
| 20 | 1.976 | 8.900 | 9.573 |
| 30 | 642 | 2.795 | 2.769 |
| 40 | 251 | 1.063 | 955 |

Die Messergebnisse in Tabelle 1 zeigen, dass die erfindungsgemäßen Verbindungen eine Erniedrigung der dynamischen Viskosität, besonders bei niedrigen Temperaturen aufweisen. Insbesondere bei Temperaturen unter 20°C ist die dynamische Viskosität des erfindungsgemäßen Reaktivharz-Masterbatches, der die erfindungsgemäße Verbindung (IV) enthält, niedriger als die dynamische Viskosität der Reaktivharz-Masterbatches C1 und D1, die die Vergleichsverbindungen 1 und 2 enthalten.

Des Weiteren wurde die dynamische Viskosität der aus dem erfindungsgemäßen Reaktivharz-Masterbatch A1 hergestellten Reaktivharzkomponente A3.1 mit der dynamischen Viskosität der aus den Vergleichsreaktivharz-Masterbatches C1 und D1 hergestellten Reaktivharzkomponenten C3.1 und D3 verglichen (Tabelle 2). Alle Reaktivharzkomponenten aus Tabelle 2 enthielten 33 Gew.-% Backbone-Harz im Reaktivharz.

**Tabelle 2: Ergebnisse der Messungen der dynamischen Viskosität der Reaktivharzkomponente A3.1 sowie der Vergleichsreaktivharzkomponenten C3.1 und D3**

| | **Reaktivharzkomponente A3.1** | **Vergleichsreaktivharzkomponente C3.1** | **Vergleichsreaktivharzkomponente D3** |
|---|---|---|---|
| **dynamische Viskosität [Pa·s]; 25°C** | 11,3 | 13,9 | 12,8 |

Die Messergebnisse in Tabelle 2 zeigen, dass die erfindungsgemäßen Verbindungen auch zu einer Erniedrigung der dynamischen Viskosität der diese enthaltenden Reaktivharzkomponenten führt. Die dynamische Viskosität der erfindungsgemäßen Reaktivharzkomponente, die die erfindungsgemäße Verbindung (IV) enthält, ist niedriger als die dynamische Viskosität der Vergleichsreaktivharzkomponenten C3.1 und D3, die die Vergleichsverbindungen 1 und 2 enthalten.

Um zu zeigen, dass es durch die Verwendung der erfindungsgemäßen Verbindungen am Beispiel der Verbindung (IV) möglich ist, den Anteil an Backbone-Harz in dem Reaktivharz und damit in der Reaktivharzkomponente zu erhöhen, ohne dass hierdurch die Viskosität zu stark ansteigt und die Auspresskräfte der zu stark ansteigen, wurde die dynamische Viskosität von Reaktivharzkomponenten mit unterschiedlich hohem Anteil an Backbone-Harz gemessen (Tabelle 3).

**Tabelle 3: Ergebnisse der Messung der dynamischen Viskosität der Reaktivharzkomponenten B3.1, B3.2, B3.3 und B3.4 sowie der Vergleichsreaktivharzkomponente C3.3**

| | **Reaktivharz komponente B3.1** | **Reaktivharz komponente B3.2** | **Reaktivharz komponente B3.3** | **Reaktivharz komponente B3.4** | **Vergleichsreaktivharzkomponente C3.3** |
|---|---|---|---|---|---|
| **Anteil Backbone-Harz im Reaktivharz** | 37 Gew.-% | 40 Gew.-% | 45 Gew.-% | 50% Gew.-% | 37 Gew.-% |
| **Dyn. Viskosität [Pa·s]; 25°C** | 7,4 | 8,8 | 11,7 | 15,1 | 12,5 |

Die Ergebnisse in Tabelle 3 zeigen, dass die dynamische Viskosität der Reaktivharzkomponente trotz Erhöhung des Anteils an Backbone-Harz relativ niedrig bleibt. Selbst eine Erhöhung des Backbone-Harzanteils auf 45 Gew.-% führt zu einer Reaktivharzkomponente, die eine niedrigere Viskosität aufweist als die Vergleichsreaktivharzkomponente mit einem Backbone-Harzanteil von 37 Gew.-%.

Sogar bei einem Backbone-Harzanteil von 50 Gew.-% liegt die Viskosität der Reaktivharzkomponente nur leicht über der der Vergleichsreaktivharzkomponente mit einem Backbone-Harzanteil von 37 Gew.-%. Bei gleichem Anteil an Backbone-Harz in dem Reaktivharz ist die dynamische Viskosität der erfindungsgemäßen Reaktivharzkomponente deutlich niedriger als die der Vergleichsreaktivharzkomponente.

### Bestimmung der Auspresskräfte

Zur Bestimmung der Auspresskräfte der Reaktivharz-Systeme wurden die Kartuschen mit den jeweiligen Reaktivharzkomponenten (Komponente (A)) und Härterkomponenten (Komponente (B)) auf 0°C bzw. 25°C temperiert. Die Kartuschen wurden auf einer Material-Prüfmaschine der Firma Zwick mit einem Kraftaufnehmer (Prüfbereich bis 10 kN) über einen Statikmischer (Mischer HIT-RE-M; Hilti Aktiengesellschaft) mit einer konstanten Geschwindigkeit von 100 mm/min über eine Strecke von 45 mm ausgepresst und die mittlere dabei auftretende Kraft gemessen.

Die Auspresskräfte des Zweikomponenten-Reaktivharz-Systems A4.1 sowie der Vergleichszweikomponenten-Reaktivharz-Systeme C4.1 und D4 mit jeweils 33 Gew.-% Backbone-Harzanteil im Reaktivharz wurden bei 0°C und 25°C gemessen (Tabelle 4).

**Tabelle 4: Ergebnisse der Messung der Auspresskräfte der Zweikomponenten-Reaktivharz-Systeme A4.1 und der Vergleichszweikomponenten-Reaktivharz-Systeme C4.1 und D4 bei 0°C und 25°C**

| | **Zweikomponenten-Reaktivharz-System A4.1** | **Vergleichszweikomponenten-Reaktivharz-System C4.1** | **Vergleichszweikomponeten-Reaktivharz-System D4** |
|---|---|---|---|
| **Kraft [N] bei 0°C** | 1322 | 1631 | 1639 |
| **Kraft [N] bei 25°C** | 1036 | 1151 | 1079 |

Die Auspresskräfte des Zweikomponenten-Reaktivharz-Systems B4.1 sowie des Vergleichszweikomponenten-Reaktivharz-Systems C4.3 mit jeweils 37 Gew.-% Backbone-Harzanteil im Reaktivharz wurden bei 0°C und 25°C gemessen (Tabelle 5).

**Tabelle 5: Ergebnisse der Messung der Auspresskräfte des Zweikomponenten-Reaktivharz-Systems B4.1 und des Vergleichszweikomponenten-Reaktivharz-Systems C4.3 bei 0°C und 25°C**

| | **Zweikomponenten-Reaktivharz-System B4.1** | **Vergleichszweikomponenten-Reaktivharz-System C4.3** |
|---|---|---|
| **Kraft [N] bei 0°C** | 579 | 700 |
| **Kraft [N] bei 25°C** | 281 | 368 |

Die Ergebnisse in den Tabellen 4 und 5 zeigen, dass die Auspresskräfte der erfindungsgemäßen Zweikomponenten-Reaktivharz-Systeme sowohl bei 0°C als auch bei 25°C geringer sind als die Auspresskräfte der Vergleichszweikomponenten-Reaktivharz-Systeme.

### Messung der Verbundspannung

Zur Bestimmung der Verbundspannungen (Lastwerte) der ausgehärteten Befestigungsmassen wurden Ankergewindestanden M12 in Bohrlöcher in Beton C20/25 mit einem Durchmesser von 14 mm und einer Bohrlochtiefe von 72 mm, die mit den Reaktionsharzmörtel-Zusammensetzungen gefüllt wurden, eingeführt. Die Verbundspannungen wurden durch zentrisches Ausziehen der Ankergewindestanden ermittelt. Es wurden jeweils fünf Ankergewindestangen gesetzt und nach 24 Stunden Aushärtung die Verbundspannung bestimmt. Die Befestigungsmassen wurden über einen Statikmischer (Mischer HIT-RE-M; Hilti Aktiengesellschaft) aus den Kartuschen ausgepresst und in die Bohrlöcher injiziert.

Die Ermittlung der Verbundspannung erfolgte bei den folgenden Bohrlochbedingungen:
A1: Es handelt sich um ein gereinigtes, staubfreies, trockenes, hammergebohrtes Bohrloch. Das Setzen, Aushärten und Ausziehen erfolgt bei Raumtemperatur. Die Temperatur der Zweikomponenten-Reaktivharz-Systeme bzw. der Befestigungsmassen beträgt beim Setzen 20°C.
F1b*: Es handelt sich um ein halbgereinigtes (ca. 50% staubfrei), feuchtes, hammergebohrtes Bohrloch. Das Setzen, Aushärten und Ausziehen erfolgt bei Raumtemperatur. Die Temperatur der Zweikomponenten-Reaktivharz-Systeme bzw. der Befestigungsmassen beträgt beim Setzen 20°C.
A21 (80°C): Es handelt sich um ein gereinigtes, staubfreies, trockenes, hammergebohrtes Bohrloch. Das Setzen und Aushärten erfolgt bei Raumtemperatur. Danach wird der Beton und Dübel 24 h bei 80°C gelagert. Das Ausziehen erfolgt bei 80°C. Die Temperatur der Zweikomponenten-Reaktivharz-Systeme bzw. der Befestigungsmassen beträgt beim Setzen 20°C.
A23 (-5°C): Es handelt sich um ein gereinigtes, staubfreies, trockenes, hammergebohrtes Bohrloch. Das Setzen, Aushärten und Ausziehen erfolgt bei -5°C. Die Temperatur der Zweikomponenten-Reaktivharz-Systeme bzw. der Befestigungsmassen beträgt beim Setzen 0°C.

Die hierbei ermittelten Verbundspannungen (N/mm²) sind als Mittelwert von fünf Messungen in den nachfolgenden Tabellen 6 und 7 aufgeführt.

**Tabelle 6: Verbundspannungen gemessen unter A1-, F1b*-, A23- und A21-Bedingungen der Befestigungsmassen aus den Zweikomponenten-Reaktivharz-Systemen A4.1 und A4.2 und der Vergleichsbefestigungsmasse aus dem Vergleichszweikomponenten-Reaktivharz-System C4.1**

| | **Verbundspannung [N/mm²]** | | | |
|---|---|---|---|---|
| | A1 | F1b* | A23 (-5°C) | A21 (80°C) |
| Befestigungsmasse aus A4.1 mit 33 Gew.-% Backbone-Harz im Reaktivharz | 18,3 | 12,3 | 10,2 | 12,9 |
| Befestigungsmasse aus A4.2 mit 41 Gew.-% Backbone-Harz im Reaktivharz | 20,9 | 14,5 | 13,5 | 14,3 |
| Vergleichsbefestigungsmasse aus C4.1 mit 33 Gew.-% Backbone-Harz im Reaktivharz | 20,3 | 13,0 | 11,6 | 11,9 |

Die Ergebnisse in Tabelle 6 zeigen, dass die Verbundspannungen der Befestigungsmasse aus A4.1, die einen Backbone-Harzanteil von 33 Gew.-% hat, unter A1, F1b* und A23-Bedingungen etwa auf demselben Niveau liegen wie die Verbundspannungen der Vergleichsbefestigungsmasse aus C4.1. Unter A21-Bedingungen sind die Verbundspannungen der Befestigungsmasse aus A4.1 etwas höher als die Verbundspannungen der Vergleichsbefestigungsmasse aus C4.1. Ferner ist ersichtlich, dass eine Erhöhung des Anteils an Backbone-Harz, wie bei der Befestigungsmasse aus A4.2 auch zu einer Erhöhung der Verbundspannungen führt.

Dies zeigen auch die Ergebnisse in Tabelle 7, wobei mit steigendem Backbone-Harzanteil (B4.1 → B4.4) eine Erhöhung der Verbundspannungen verglichen mit der Verbundspannung der Vergleichsbefestigungsmasse aus C4.3 (37 Gew.-% Backbone-Harz im Reaktivharz) beobachtet wurde.

**Tabelle 7: Ergebnisse der Messungen der Verbundspannungen, gemessen unter A1-Bedingungen, der Befestigungsmassen aus den Zweikomponenten-Reaktivharz-Systemen B4.1 bis B4.4 und der Vergleichsbefestigungsmasse aus dem Vergleichszweikomponenten-Reaktivharz-System C4.3**

| **Befestigungsmasse aus** | **B4.1** | **B4.2** | **B4.3** | **B4.4** | **C4.3** |
|---|---|---|---|---|---|
| Anteil Backbone-Harz im Reaktivharz | 37 Gew.-% | 40 Gew.-% | 45 Gew.-% | 50 Gew.-% | 37 Gew.-% |
| Verbundspannung [N/mm²] | 33,7 | 34,4 | 34,6 | 35,6 | 33,3 |

Ferner wurden Reaktivharze, Reaktivharzkomponenten und Zweikomponenten-Reaktivharz-Systeme, die jeweils die erfindungsgemäße Verbindung (IV) als Backbone-Harz enthielten, hergestellt. Die dynamische Viskosität der Reaktivharze und der Reaktivharzkomponenten sowie die Auspresskräfte der Zweikomponenten-Reaktivharz-Systeme wurden ermittelt.

### Erfindungsgemäße Verbindung (V)

Ferner wurden Reaktivharz-Masterbatches, Reaktivharze, Reaktivharzkomponenten und Zweikomponenten-Reaktivharz-Systeme, die jeweils die erfindungsgemäße Verbindung (V) als Backbone-Harz enthielten, hergestellt. Die dynamische Viskosität der Reaktivharz-Masterbatches und der Reaktivharzkomponenten sowie die Auspresskräfte der Zweikomponenten-Reaktivharz-Systeme wurden ermittelt und mit den entsprechenden Werten für die Vergleichszusammensetzungen verglichen.

### E1.1 Herstellung des Reaktivharz-Masterbatches E1.1 mit 65 Gew.-% Verbindung (V) und 35 Gew.-% 1,4-Butandioldimethacrylat

In einem 300 Liter Stahlreaktor mit Innenthermometer und Rührwelle wurden 80400 g Hydroxypropylmethacrylat (Visiomer^{®} HPMA; Evonik Degussa GmbH) vorgelegt und mit 36 g Phenothiazin (D Prills; Allessa Chemie), 70 g 4-Hydroxy-2,2,6,6-tetramethyl-piperidinyl-1-oxyl (TEMPOL; Evonik Degussa GmbH) und 56 g Dioctylzinndilaurat (TIB KAT® 216; TIB Chemicals) versetzt. Der Ansatz wurde auf 60°C erwärmt. Anschließend wurden 69440 g Methylen-di(phenylisocyanat) (MDI; Lupranat^{®} MIS, BASF SE) unter Rühren über 1,5 h zugetropft. Danach wurde weitere 45 Minuten bei 80°C gerührt. Anschließend wurden 50000 g 1,4-Butandioldimethacrylat (Visiomer 1,4-BDDMA, Evonik Degussa GmbH) zugegeben.

Es wurde der Reaktivharz-Masterbatch E1.1 erhalten, der 75 Gew.-% der Verbindung (V) als Backbone-Harz und 25 Gew.-% 1,4-Butandioldimethacrylat, bezogen auf das Gesamtgewicht des Reaktivharz-Masterbatches, enthielt.

Durch Verdünnung mit 1,4-Butandioldimethacrylat konnte der Reaktivharz-Masterbatch E1.1 soweit verdünnt werden, dass der Masterbatch 65 Gew.-% Verbindung (V) und 35 Gew.-% 1,4-Butandioldimethacrylat enthielt.

Die Verbindung (V) weist die folgende Struktur auf:

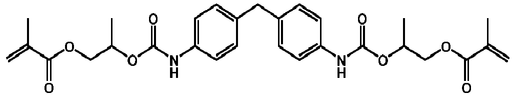

### E1.2 Herstellung des erfindungsgemäßen Reaktivharz-Masterbatches E1.2 mit 65 Gew.-% Verbindung (V) und 35 Gew.-% Hydroxypropylmethacrylat

In einem 2 Liter Laborglasreaktor mit Innenthermometer und Rührwelle wurden 1396 g Hydroxypropylmethacrylat (Visiomer HPMA 98; Evonik Degussa GmbH) vorgelegt und mit 0,3 g Phenothiazin (D Prills; Allessa Chemie), 0,6 g 4-Hydroxy-2,2,6,6-tetramethyl-piperidinyl-1-oxyl (TEMPOL; Evonik Degussa GmbH) und 0,48 g Dioctylzinndilaurat (TIB KAT^{®} 216; TIB Chemicals) versetzt. Der Ansatz wurde auf 60 °C erwärmt. Anschließend wurden 602,6 g Methylen-di(phenylisocyanat) (MDI; Lupranat^{®} MIS, BASF SE) unter Rühren (600 U/min) über 1,5 h zugetropft. Danach wurde weitere 30 Minuten bei 80°C gerührt.

Hierdurch wurde der Reaktivharz-Masterbatch E1.2 erhalten, der 65 Gew.-% der Verbindung (V) als Backbone-Harz und 35 Gew.-% Hydroxypropylmethacrylat, bezogen auf das Gesamtgewicht des Reaktivharz-Masterbatches, enthielt.

### E2. Herstellung des Reaktivharzes E2 mit 45 Gew.-% Verbindung (V)

2520 g Reaktivharz-Masterbatch aus E1.1 wird mit 439,74 g Hydroxypropylmethacrylat und 1128,54 g 1,4-Butandioldimethacrylat (1,4-BDDMA; Evonik Degussa GmbH) gemischt. Zu dieser Mischung wurden 96,6 g Di-isopropanol-p-toluidin (BASF SE), 13,44 g Catechol (Catechol Flakes, RHODIA) und 5,88 g *tert*-Butylbrenzkatechin (tBBK, CFS EUROPE S.p.A. (Borregaard Italia S.p.A.)) gegeben und bis zur vollständigen Homogenisierung gerührt.

Hierdurch wurde das Reaktivharz E2 mit 45 Gew.-% der Verbindung (V) als Backbone-Harz erhalten.

### E3.1 Herstellung der Reaktivharzkomponente E3.1

### (für die Messung der Viskosität und der Auspresskräfte bei 23°C)

2106 g Reaktivharz E2 werden mit 930,42 g Secar^{®} 80 (Kerneos Inc.), 64,8 g Cab-O-Sil^{®} TS-720 (Cabot Corporation), 90,72 g Aerosil^{®} R 812 (Evonik Industries AG) und 2222,64 g Quarzsand F32 (Quarzwerke GmbH) im Dissolver mit einem PC Laborsystem Dissolver vom Typ LDV 0.3-1 unter Vakuum vermischt. Die Mischung wurde für 2 Minuten bei 2500 U-min⁻¹ und danach für 10 Minuten bei 4500 U·min⁻¹ unter Vakuum (Druck≤100 mbar) mit einer 55 mm Dissolverscheibe und einem Randabstreifer gerührt.

Hierdurch wurde die Reaktivharzkomponente E3.1 erhalten.

### E3.2 Herstellung der Reaktivharzkomponente E3.2

### (für die Messung der Viskosität bei 0°C und 25°C und der Auspresskräfte bei 0°C)

1053 g Reaktivharz E2 werden mit 465,21 g Secar^{®} 80 (Kerneos Inc.), 27 g Cab-O-Sil^{®} TS-720 (Cabot Corporation), 48,6 g Aerosil^{®} R 812 (Evonik Industries AG) und 1111,32 g Quarzsand F32 (Quarzwerke GmbH) im Dissolver unter Vakuum vermischt. Das Mischen erfolgte mit einem PC Laborsystem Dissolver vom Typ LDV 0.3-1, wie unter Punkt E3.1 beschrieben.

Hierdurch wurde die Reaktivharzkomponente E3.2 erhalten.

### E4. Herstellung der Zweikomponenten-Reaktivharz-Systeme E4.1 und E4.2

Zur Herstellung der Zweikomponenten-Reaktivharz-Systeme E4.1 und E4.2 wurde jeweils die Reaktivharzkomponente E3.1 bzw. E3.2 (Komponente (A)) und die Härterkomponente (Komponente (B)) des kommerziell erhältlichen Produkts HIT-HY 200 (Hilti Aktiengesellschaft; Chargennummer: 8104965) in eine Plastikkartusche (Firma Ritter GmbH; Volumenverhältnis A:B = 5:1) mit den Innendurchmessern 32,5 mm (Komponente (A)) bzw. 14 mm (Komponente (B)) gefüllt.

Hierdurch wurden die Zweikomponenten-Reaktivharz-Systeme E4.1 (für die Messung der der Auspresskräfte bei 23°C) und E4.2 (für die Messung der der Auspresskräfte bei 0°C) erhalten.

### Vergleichsbeispiele F und G

Zum Vergleich wurden Reaktivharz-Masterbatches, Reaktivharze und Reaktivharzkomponenten mit den Vergleichsverbindungen 1 und 2 wie folgt hergestellt.

### F1. Herstellung der Vergleichsreaktivharz-Masterbatches F1.1 und F1.2

Ein Vergleichsreaktivharz-Masterbatch mit 65 Gew.-% der Vergleichsverbindung 1 als Backbone-Harz und 35 Gew.-% 1,4-Butandioldimethacrylat (F1.1) bzw. Hydroxypropylmethacrylat (F1.2), jeweils bezogen auf das Gesamtgewicht des Reaktivharz-Masterbatches, wurde gemäß dem Verfahren in EP 0 713 015 A1, die hiermit als Referenz eingeführt und auf deren gesamte Offenbarung verwiesen wird, synthetisiert.

Das Produkt (Vergleichsverbindung 1) hat eine Oligomerverteilung, wobei das Oligomer mit einer Wiederholungseinheit folgende Struktur hat:

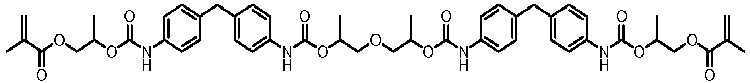

### F2. Herstellung des Vergleichsreaktivharzes F2 mit 45 Gew.-% Vergleichsverbindung 1

830,76 g Vergleichsreaktivharz-Masterbatch F1.1 wurden mit 125,64 g Hydroxypropylmethacrylat und 211,68 g 1,4-Butandioldimethacrylat (1,4-BDDMA; Evonik Degussa GmbH) gemischt. Zu dieser Mischung wurden 27,6 g Di-isopropanol-p-toluidin (BASF SE), 3,24 g Catechol (Catechol Flakes, RHODIA) und 1,08 g *tert-*Butylbrenzkatechin (tBBK, CFS EUROPE S.p.A. (Borregaard Italia S.p.A.)) gegeben und bis zur vollständigen Homogenisierung gerührt.

Hierdurch wurde das Vergleichsreaktivharz F2 mit 45 Gew.-% Anteil an Vergleichsverbindung 1 als Backbone-Harz erhalten.

### F3.1. Herstellung der Vergleichsreaktivharzkomponente F3.1

### (für die Messung der Viskosität und der Auspresskräfte bei 23°C)

1053 g Vergleichsreaktivharz F2 wurden mit 465,21 g Secar^{®} 80 (Kerneos Inc.), 32,4 g Cab-O-Sil^{®} TS-720 (Cabot Corporation), 45,36 g Aerosil^{®} R 812 (Evonik Industries AG) und 1111,33 g Quarzsand F32 (Quarzwerke GmbH) im Dissolver unter Vakuum vermischt.

Hierdurch wurde die Vergleichsreaktivharzkomponente F3.1 mit der Vergleichsverbindung 1 als Backbone-Harz erhalten.

### F3.2. Herstellung der Vergleichsreaktivharzkomponente F3.2

### (für die Messung der Viskosität bei 0°C und 25°C und der Auspresskräfte bei 0°C)

1053 g Vergleichsreaktivharz F2 wurden mit 465,21 g Secar^{®} 80 (Kerneos Inc.), 27 g Cab-O-Sil^{®} TS-720 (Cabot Corporation), 48,6 g Aerosil^{®} R 812 (Evonik Industries AG) und 1111,32 g Quarzsand F32 (Quarzwerke GmbH) im Dissolver unter Vakuum vermischt.

Hierdurch wurde die Vergleichsreaktivharzkomponente F3.2 mit der Vergleichsverbindung 1 als Backbone-Harz erhalten.

### F4. Herstellung der Vergleichszweikomponenten-Reaktivharz-Systeme F4.1 und F4.2

Zur Herstellung der Zweikomponenten-Reaktivharz-Systeme F4.1 und F4.2 wurde jeweils die Reaktivharzkomponente F3.1 bzw. F3.2 (Komponente (A)) und die Härterkomponente (Komponente (B)) des kommerziell erhältlichen Produkts HIT-HY 200 (Hilti Aktiengesellschaft; Chargennummer: 8104965) in eine Plastikkartusche (Firma Ritter GmbH; Volumenverhältnis A:B = 5:1) mit den Innendurchmessern 32,5 mm (Komponente (A)) bzw. 14 mm (Komponente (B)) gefüllt.

Hierdurch wurden die Zweikomponenten-Vergleichsreaktivharz-Systeme F4.1 (für die Messung der Auspresskräfte bei 23°C) und F4.2 (für die Messung der Auspresskräfte bei 0°C) erhalten.

### G1. Herstellung der Vergleichsreaktivharz-Masterbatches G1.1 und G1.2

Die Vergleichsreaktivharz-Masterbatches G1.1 und G1.2, jeweils mit 65 Gew.-% Referenzverbindung 2 als Backbone-Harz und 35 Gew.-% 1,4-Butandiolodimethacrylat (G1.1) bzw. Hydroxypropylmethacrylat (G1.2), jeweils bezogen auf das Gesamtgewicht des Reaktivharz-Masterbatches, wurden gemäß dem Verfahren in EP 0 713 015 A1, die hiermit als Referenz eingeführt und auf deren gesamte Offenbarung verwiesen wird, synthetisiert.

Die Vergleichsverbindung 2 hat folgende Struktur:

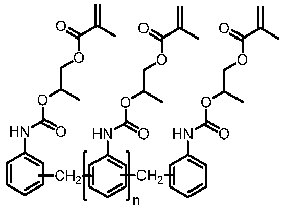

### G2. Herstellung des Vergleichsreaktivharzes G2 mit 45 Gew.-% Vergleichsverbindung 2

830,76 g Vergleichsreaktivharz-Masterbatch G1.1 wurden mit 125,64 g Hydroxypropylmethacrylat und 211,68 g 1,4-Butandioldimethacrylat (1,4-BDDMA; Evonik Degussa GmbH) gemischt. Zu dieser Mischung wurden 27,6 g Di-isopropanol-p-toluidin (BASF SE), 3,24 g Catechol (Catechol Flakes, RHODIA) und 1,08 g tert-Butylbrenzkatechin (tBBK, CFS EUROPE S.p.A. (Borregaard Italia S.p.A.)) gegeben und bis zur vollständigen Homogenisierung gerührt.

Hierdurch wird das Vergleichsreaktivharz G2 mit 45 Gew.-% Anteil an Verbindung 2 als Backbone-Harz in Hydroxypropylmethacrylat und 1,4-Butandioldimethacrylat erhalten.

### G3.1. Herstellung der Vergleichsreaktivharzkomponente G3.1

### (für die Messung der Viskosität und der Auspresskräfte bei 23°C)

1053 g Vergleichsreaktivharz G2 wurden mit 465,21 g Secar^{®} 80, 32,4 g Cab-O-Sil^{®} TS-720 (Cabot Corporation), 45,36 g Aerosil^{®} R 812 (Evonik Industries AG) und 1111,32 g Quarzsand F32 (Quarzwerke GmbH) im Dissolver unter Vakuum vermischt.

Hierdurch wurde die Vergleichsreaktivharzkomponente G3.1 mit der Vergleichsverbindung 2 als Backbone-Harz erhalten.

### G3.2. Herstellung der Vergleichsreaktivharzkomponente G3.2

### (für die Messung der Viskosität bei 0°C und 25°C und der Auspresskräfte bei 0°C)

1053 g Vergleichsreaktivharz G2 wurden mit 465,21 g Secar^{®} 80 (Kerneos Inc.), 27 g Cab-O-Sil^{®} TS-720 (Cabot Corporation), 48,6 g Aerosil^{®} R 812 (Evonik Industries AG) und 1111,32 g Quarzsand F32 (Quarzwerke GmbH) im Dissolver unter Vakuum vermischt.

Hierdurch wurde die Vergleichsreaktivharzkomponente G3.2 mit der Vergleichsverbindung 2 als Backbone-Harz erhalten.

### G4. Herstellung der Vergleichszweikomponenten-Reaktivharz-Systeme G4.1 und G4.2

Zur Herstellung der Vergleichszweikomponenten-Reaktivharz-Systeme G4.1 und G4.2 wurde jeweils die Reaktivharzkomponente G3.1 bzw. G3.2 (Komponente (A)) und die Härterkomponente (Komponente (B)) des kommerziell erhältlichen Produkts HIT-HY 200 (Hilti Aktiengesellschaft; Chargennummer: 8104965) in eine Plastikkartusche (Firma Ritter GmbH; Volumenverhältnis A:B = 5:1) mit den Innendurchmessern 32,5 mm (Komponente (A)) bzw. 14 mm (Komponente (B)) gefüllt.

Hierdurch wurden die Zweikomponenten-Vergleichsreaktivharz-Systeme G4.1 (für die Messung der Auspresskräfte bei 23°C) und G4.2 (für die Messung der Auspresskräfte bei 0°C) erhalten.

Um den Einfluss der erfindungsgemäßen Verbindung (V) auf die Viskosität eines diese enthaltenden Reaktivharz-Masterbatches, eines Reaktivharzes und einer Reaktivharzkomponente zu zeigen, wurden die Viskosität der erfindungsgemäßen Reaktivharz-Masterbatches, Reaktivharze, Reaktivharzkomponenten sowie die Auspresskräfte von Reaktivharz-Systemen gemessen und jeweils mit den Vergleichsformulierungen verglichen.

### Messung der dynamischen Viskosität der Reaktivharz-Masterbatches E1.1 und E1.2 sowie der Vergleichsreaktivharz-Masterbatches F1.1, F1.2, G1.1 und G1.2

Die Messung der dynamischen Viskosität der Reaktivharz-Masterbatches E1.1 und E1.2 und der Vergleichsreaktivharz-Masterbatches F1.1, F1.2, G1.1 und G1.2 (Tabelle 8) erfolgte mit einem Kegel-Platte Messsystem nach DIN 53019. Der Durchmesser des Kegels betrug 20mm und der Öffnungswinkel war 1°. Es wurde bei einer konstanten Schergeschwindigkeit von 100/s bei der jeweiligen Temperatur gemessen (0, 5, 10, 15, 20, 30 und 40°C). Die Messdauer betrug 120s und jede Sekunde wurde ein Messpunkt generiert. Zur Erreichung der Schergeschwindigkeit bei der jeweiligen Temperatur wurde eine Rampe von 0-100/s mit einer Dauer von 30s vorgeschaltet. Da es sich um newtonschen Flüssigkeiten handelt, wurde über den Messabschnitt mit konstanter Schergeschwindigkeit von 100/s eine lineare Auswertung über den Messabschnitt vorgenommen und die Viskosität bestimmt. Es wurden jeweils drei Messungen gemacht, wobei in Tabelle 8 unten die entsprechenden Mittelwerte angegeben sind.

### Messung der dynamischen Viskosität der Reaktivharzkomponente E3.1 sowie der Vergleichsreaktivharzkomponenten F3.1 und G3.1

Die Messung der dynamischen Viskosität der Reaktivharzkomponente E3.1 sowie der Vergleichsreaktivharzkomponenten F3.1 und G3.1 (Tabelle 9) erfolgte mit einem Platte-Platte Messsystem nach DIN 53019. Der Durchmesser der Platte betrug 35mm und der Spaltabstand betrug 3mm. Um ein Austreten der Probe aus dem Spalt zu verhindern, wurde ein Begrenzungsring aus Teflon verwendet, der einen Abstand von 1 mm von der oberen Platte hatte. Die Messtemperatur betrug 23°C. Die Methode bestand aus zwei Abschnitten: 1. Eine Rampe von 0/s bis 10/s mit einer Dauer von 120s mit 100 Messpunkten. 2. Konstante Scherung mit 10/s für 180s mit 180 Messpunkten. Von dem zweiten Abschnitt wurde eine lineare Auswertung vorgenommen und der Wert als Viskosität angegeben. Es wurden jeweils drei Messungen gemacht, wobei in Tabelle 9 die entsprechenden Mittelwerte angegeben sind.

### Messung der dynamischen Viskosität der Reaktivharzkomponente E3.2 sowie der Vergleichsreaktivharzkomponenten F3.2 und G3.2

Die Messung der dynamischen Viskosität der Reaktivharzkomponente E3.2 sowie der Vergleichsreaktivharzkomponenten F3.2 und G3.2 (Tabelle 10) erfolgte mit einem Platte-Platte Messsystem nach DIN 53019. Der Durchmesser der Platte betrug 20mm und der Spaltabstand betrug 3mm. Um ein Austreten der Probe aus dem Spalt zu verhindern, wurde ein Begrenzungsring aus Teflon verwendet, der einen Abstand von 1mm von der oberen Platte hatte. Die Messtemperatur betrug 0°C bzw. 25°C. Die Methode bestand aus drei Abschnitten: 1. Niedrige Scherung, 2. Hohe Scherung, 3. Niedrige Scherung. Beim 1. Abschnitt wurde 3 Minuten bei 0,5/s geschert. Im 2. Abschnitt wurde in 8 Stufen zu je 15 Sekunden logarithmisch die Schergeschwindigkeit von 0,8/s auf 100/s gesteigert. Die einzelnen Stufen dabei waren: 0,8/s; 1,724/s; 3,713/s; 8/s; 17,24/s; 37,13/s; 80/s; 100/s. Der 3. Abschnitt war die Wiederholung des 1 Abschnitts. Am Ende eines jeden Abschnittes wurden die Viskositäten ausgelesen. In Tabelle 10 sind die Werte des zweiten Abschnittes bei 8/s und 100/s angegeben. Es wurden jeweils drei Messungen gemacht, wobei in Tabelle 10 die entsprechenden Mittelwerte angegeben sind.

Zunächst wurde die dynamische Viskosität des Reaktivharz-Masterbatches E1.1 und E1.2 mit den Vergleichsreaktivharz-Masterbatches F1.1, F1.2, G1.1 und G1.2 bei unterschiedlichen Temperaturen verglichen (Tabelle 8). Die Reaktivharz-Masterbatches enthielten jeweils 65 Gew.-% Backbone-Harz und 35 Gew.-% Hydroxypropylmethacrylat (E1.1, F1.1, G1.1) bzw. 35 Gew.-% 1,4-Butandiodimethacrylat (E1.2, F1.2, G1.2).

**Tabelle 8: Ergebnisse der Messungen der dynamischen Viskosität des Reaktivharz-Masterbatches E1.1 und E1.2 sowie der Vergleichsreaktivharz-Masterbatches F1.1, F1.2, G1.1 und G1.2 bei unterschiedlichen Temperaturen**

| | **E1.2** | **E1.1** | **F1.2** | **F1.1** | **G1.2** | **G1.1** |
|---|---|---|---|---|---|---|
| **T [°C]** | **dynamische Viskosität [mPa·s]** | | | | | |
| **40** | 283 | 301 | 952 | 599 | 961 | 1.147 |
| **30** | 736 | 737 | 2.897 | 1.579 | 2.877 | 3.139 |
| **20** | 2.356 | 2.159 | 11.045 | 5.045 | 10.745 | 10.475 |
| **15** | 4.716 | 4.104 | 24.505 | 10.051 | 23.240 | 21.145 |
| **10** | 10.270 | 8.387 | 59.360 | 21.480 | 54.960 | 45.855 |
| **5** | 24.150 | 18.435 | 156.750 | 49.575 | 137.250 | 106.650 |
| **0** | 62.355 | 43.800 | 438.800 | 123.350 | 339.950 | 265.200 |

Die Messergebnisse zeigen, dass die erfindungsgemäßen Reaktivharz-Masterbatches eine Verringerung der dynamischen Viskosität, besonders bei niedrigen Temperaturen, aufweisen. Insbesondere bei Temperaturen unter 20°C liegt die dynamische Viskosität des erfindungsgemäßen Reaktivharz-Masterbatches mit der Verbindung (V) als Backbone-Harz deutlich unter der Viskosität der Vergleichsreaktivharz-Masterbatches mit den Vergleichsverbindungen 1 und 2.

Des Weiteren wurde die dynamische Viskosität der aus dem erfindungsgemäßen Reaktivharz-Masterbatch E1.1 hergestellten Reaktivharzkomponente E3.1 mit der dynamischen Viskosität der aus den Vergleichsreaktivharz-Masterbatches F1.1 und G1.1 hergestellten Vergleichsreaktivharzkomponenten F3.1 und G3.1 verglichen (Tabelle 9). Alle in Tabelle 9 gezeigten Reaktivharzkomponenten enthielten 45 Gew.-% Backbone-Harz im Reaktivharz.

**Tabelle 9: Ergebnisse der Messung der dynamischen Viskosität der Reaktivharzkomponente E3.1 sowie der Vergleichsreaktivharzkomponenten F3.1 und G3.1**

| | **E3.1** | **F3.1** | **G3.1** |
|---|---|---|---|
| **Dyn. Viskosität [Pa·s]; 23°C** | 22,9 | 26,3 | 32,5 |

Die Ergebnisse in Tabelle 9 zeigen, dass die dynamische Viskosität der Reaktivharzkomponente, die die erfindungsgemäße Verbindung (V) enthält, relativ niedrig ist, verglichen mit der dynamischen Viskosität der Vergleichsreaktivharzkomponenten, die die Vergleichsverbindungen 1 bzw. 2 enthalten.

Um Auszuschließen, dass die Unterschiede in der dynamischen Viskosität der Reaktivharzkomponenten von der verwendeten Kieselsäure-Zusammensetzung herrühren, wurden die Messungen mit jeweils veränderten Anteilen an Kieselsäure (Reaktivharzkomponente E3.2 und Vergleichsreaktivharzkomponenten F3.2. und G3.2) und bei zwei unterschiedlichen Scherraten (8 s⁻¹ und 100 s⁻¹) und zwei Temperaturen (0°C und 25°C) wiederholt. Die Ergebnisse sind in Tabelle 10 gezeigt.

**Tabelle 10: Ergebnisse der Messung der dynamischen Viskosität der Reaktivharzkomponente E3.2 sowie der Vergleichsreaktivharzkomponenten F3.2 und G3.2**

| | **E3.2 (25 °C)** | **F3.2 (25 °C)** | **G3.2 (25 °C)** | **E3.2 (0°C)** | **F3.2 (0°C)** | **G3.2 (0°C)** |
|---|---|---|---|---|---|---|
| **Scherrate** | **dynamische Viskosität [Pa·s]** | | | | | |
| 8 s⁻¹ | 30,9 | 40,4 | 44,0 | 100,5 | 142,6 | 168,7 |
| 100 s⁻¹ | 8,3 | 11,2 | 13,2 | 36,4 | 49,6 | 61,2 |

Die Ergebnisse in Tabelle 10 zeigen, dass trotz veränderter Kieselsäure-Zusammensetzung die dynamische Viskosität der Reaktivharzkomponente E3.2, die die erfindungsgemäße Verbindung (V) enthält, sowohl bei 0°C als auch bei 25°C relativ niedrig ist, verglichen mit der dynamischen Viskosität der Vergleichsreaktivharzkomponenten F3.2 und G3.2, die die Vergleichsverbindungen 1 bzw. 2 enthalten.

### Bestimmung der Auspresskräfte

Zur Bestimmung der Auspresskräfte bei 0°C und 23°C wurden die Reaktivharz-Systeme E4.1 sowie die Vergleichsreaktivharz-Systeme F4.1 und G4.1 auf 0°C bzw. 23°C temperiert. Die Kartuschen wurden auf einer Material-Prüfmaschine der Firma Zwick mit einem Kraftaufnehmer (Prüfbereich bis 10 kN) über einen Statikmischer (Mischer HIT-RE-M; Hilti Aktiengesellschaft) mit einer konstanten Geschwindigkeit von 100 mm/min über eine Strecke von 45 mm ausgepresst und die dabei auftretende mittlere Kraft gemessen.

Die Auspresskräfte des Zweikomponenten-Reaktivharz-Systems E4.1, das die erfindungsgemäße Verbindung (V) enthält, wurden mit der Auspresskraft der Vergleichszweikomponenten-Reaktivharz-Systeme F4.1 und G4.1, die die Vergleichsverbindungen 1 bzw. 2 enthalten, bei 0°C und bei 23°C verglichen. Die Messergebnisse sind in Tabelle 11 zusammengefasst.

**Tabelle 11: Ergebnisse der Messung der Auspresskräfte des Zweikomponenten-Reaktivharz-System E4.1 und der Vergleichszweikomponenten-Reaktivharz-Systeme F4.1 und G4.1 bei 0°C und 23°C**

| | **Zweikomponentenreaktivharz-System E4.1** | **Vergleichszweikomponenten-Reaktivharz-System F4.1** | **Vergleichszweikomponenten-Reaktivharz-System G4.1** |
|---|---|---|---|
| **Kraft [N] bei 0°C** | 462,1 | 750,2 | 740,5 |
| **Kraft [N] bei 23°C** | 377,5 | 400,7 | 396,0 |

Die Ergebnisse in Tabelle 11 zeigen, dass das Zweikomponenten-Reaktivharz-System, das die erfindungsgemäße Verbindung (V) enthält, sowohl bei 0°C als auch bei 23°C eine niedrigere Auspresskraft aufweist als die Vergleichszweikomponenten-Reaktivharz-Systeme, welche die Vergleichsverbindungen 1 bzw. 2 enthalten, wobei die Unterschiede bei 0°C besonders deutlich sind.

Dies belegt, dass die erfindungsgemäßen Verbindungen zu einer Erniedrigung der Viskosität von Reaktivharz-Masterbatches und damit der entsprechenden Reaktivharze führen. Auch die daraus hergestellten Reaktivharzkomponenten haben eine verringerte Viskosität, was sich in einer Erniedrigung der Auspresskräfte widerspiegelt.

Neben der Erniedrigung der Viskosität führt die Verwendung der erfindungsgemäßen Verbindungen zu einer Erhöhung der Lastwerte der ausgehärteten Befestigungsmassen.

## Patentansprüche

1. Verbindung der allgemeinen Formel (I) 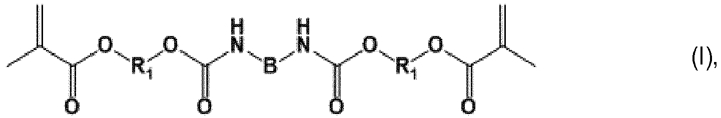 worin
B eine aromatische Kohlenwasserstoffgruppe ist, und
jedes R₁ unabhängig voneinander eine verzweigte oder lineare aliphatische C₁-C₁₅-Alkylengruppe ist.

2. Verbindung nach Anspruch 1, wobei B eine aromatische C₆-C₂₀-Kohlenstoffgruppe ist.

3. Verbindung nach Anspruch 2, wobei B einen oder zwei Benzolringe, die gegebenenfalls substituiert sind, enthält.

4. Verbindung nach einem der Ansprüche 1 bis 3, wobei R₁ eine C₂- oder C₃-Alkylengruppe ist.

5. Verwendung einer Verbindung nach einem der vorhergehenden Ansprüche zur Herstellung eines Reaktivharzes oder einer Reaktivharzkomponente für bauliche Zwecke.

6. Verwendung einer Verbindung nach einem der Ansprüche 1 bis 4 zur Erniedrigung der Viskosität eines Reaktivharzes oder zur Erniedrigung der Auspresskräfte einer Reaktivharzkomponente oder eine Reaktivharz-Systems für bauliche Zwecke.

7. Verwendung einer Verbindung nach einem der Ansprüche 1 bis 4 zur Erhöhung der Verbundspannung einer ausgehärteten Befestigungsmasse.

8. Reaktivharz umfassend eine Verbindung nach einem der Ansprüche 1 bis 4, einen Inhibitor, einen Beschleuniger und einen Reaktivverdünner.

9. Reaktivharzkomponente für ein Reaktivharz-System umfassen ein Reaktivharz nach Anspruch 8.

10. Reaktivharz-System mit einer Reaktivharzkomponente (A) nach Anspruch 9 und einer Härterkomponente (B), die einen Initiator enthält.

11. Reaktivharz-System nach Anspruch 10, wobei mindestens eine der Komponenten (A) oder (B) einen anorganischen Zuschlagstoff enthält.

12. Verwendung eines Reaktivharzes nach Anspruch 10 oder eines Reaktivharz-Systems nach Anspruch 10 oder 11 für bauliche Zwecke.

13. Verwendung nach Anspruch 12 zur chemischen Befestigung von Verankerungsmitteln in Bohrlöchern.
